# EUROPEAN PATENT SPECIFICATION

(11) **EP 3 914 734 B1**
(45) Date of publication and mention of the grant of the patent: **13.03.2024**
(21) Application number: 20770552.6
(22) Date of filing: 24.01.2020
(51) Int. Cl.: C12Q 1/686, C12Q 1/6806, C12Q 1/6853

(54) **COMPOSITIONS AND METHOD FOR SYNTHESIZING NUCLEIC ACIDS**
ZUSAMMENSETZUNGEN UND VERFAHREN ZUR SEQUENZIERUNG VON NUKLEINSÄUREN
COMPOSITIONS ET PROCÉDÉ DE SYNTHÈSE D'ACIDES NUCLÉIQUES

(30) Priority: 25.01.2019 US 201962796982 P; 09.05.2019 US 201962845539 P
(43) Date of publication of application: 01.12.2021
(73) Proprietor: President and Fellows of Harvard College, Cambridge, MA 02138 (US)
(72) Inventor: KISHI, Jocelyn, Boston, MA 02215 (US); LIU, Ninning, Boston, MA 02215 (US); SAKA, Sinem, Allston, MA 02134 (US); YIN, Peng, Brookline, MA 02445 (US)
(74) Representative: Brand Murray Fuller LLP
(86) International application number: PCT/US2020/014952
(87) International publication number: WO 2020/185312

(56) References cited:
- WO-A1-2017/143006
- WO-A2-2018/175296
- JOCELYN Y. KISHI ET AL: "Programmable autonomous synthesis of single-stranded DNA", NATURE CHEMISTRY, vol. 10, no. 2, 6 November 2017 (2017-11-06), pages 155-164, XP055622591, London ISSN: 1755-4330, DOI: 10.1038/nchem.2872
- SUN BO ET AL: "T7 replisome directly overcomes DNA damage", NATURE COMMUNICATIONS, vol. 6, no. 1, 1 December 2015 (2015-12-01), XP055957737, DOI: 10.1038/ncomms10260 Retrieved from the Internet: URL:http://www.nature.com/articles/ncomms1 0260>

## Description

### CROSS-REFERENCE TO RELATED APPLICATIONS

This application claims benefit under 35 U.S.C. § 119(e) of U.S. Provisional Application No. 62/796,982, filed January 25, 2019, and U.S. Provisional Application No. 62/845,539, filed May 9, 2019.

### GOVERNMENT SUPPORT

This invention was made with government support under 1317291 and 1729397 awarded by the National Science Foundation and GM133052 awarded by the National Institute of Health, and under N00014-16-1-2410 and N00014-18-1-2549 awarded by the Department of Defense Office of Naval Research. The government has certain rights in the invention.

### TECHNICAL FIELD

The present disclosure relates to nucleic acid templates and methods for synthesis of nucleic acids using strand displacing polymerases.

### BACKGROUND

The ability to synthesize arbitrary sequences of nucleic acids, in particular DNA, has revolutionized how scientists can study and engineer biology. Whole genomes can now be sequenced effectively, but the technology to synthesize genomes has lagged behind. Synthetic single-stranded sequences (`oligos') are typically synthesized chemically through cyclic coupling steps. However, oligonucleotides are typically not chemically synthesized past two hundred bases, due to the limitations on chemical coupling efficiency. Therefore, synthesis methods have been developed to assemble larger fragments of single-stranded and double-stranded sequences, including enzymatic assembly using sequential or simultaneous combination of multiple enzyme activities (such as restriction enzyme digestion and ligation, or isothermal Gibson assembly which combines 5' exonuclease, the 3' extension activity of a DNA polymerase and DNA ligase activity), non-enzymatic twin primer assembly, and chemical assembly (such as click DNA assembly). While these can be effective at producing longer sequences of single-stranded and double-stranded DNA, sequences of hundreds of thousands of bases must be further assembled from shorter fragments *in vivo* using yeast vectors. A new simple and robust synthesis method capable of generating long sequences of DNA could have important enabling applications in molecular biology, genome engineering, nanotechnology, and polymer-based data storage.

Kishi et al., "Programmable autonomous synthesis of single-stranded DNA Nature Chemistry 10:2 (2017) relates to primer exchange reaction (PER) cascades.

WO2018/175296 relates to methods and compositions for exponential amplification of single- and double-stranded DNA under isothermal conditions.

Thus, there remains a need in the art for compositions and methods for synthesizing nucleic acids of arbitrary length prescribed sequences, polymeric sequences of prescribed length, or sequences comprising prescribed number of repeats. The present disclosure addresses some of these needs.

### SUMMARY

Provided herein are nucleic acid templates and methods for synthesizing strands of nucleic acid across different nucleic acid backbones hybridized together using a strand displacing polymerase. The compositions and methods described herein for synthesizing strands across nucleic acid junctions can effectively decouple the assembly step of nucleic acid template from the synthesis step. This provides, among other things, the ability to sequentially perform robust annealing of guide strands, that can hybridize in predictable ways close to thermodynamic optimum, and follow up with polymerization to create the new transcript. The compositions and methods described herein can allow scaling to very long and arbitrary sequences. Moreover, the entire assembly and synthesis process can happen isothermally, including at room temperature, which enable a generalized workflow for synthesis of long arbitrary sequences under relatively mild conditions.

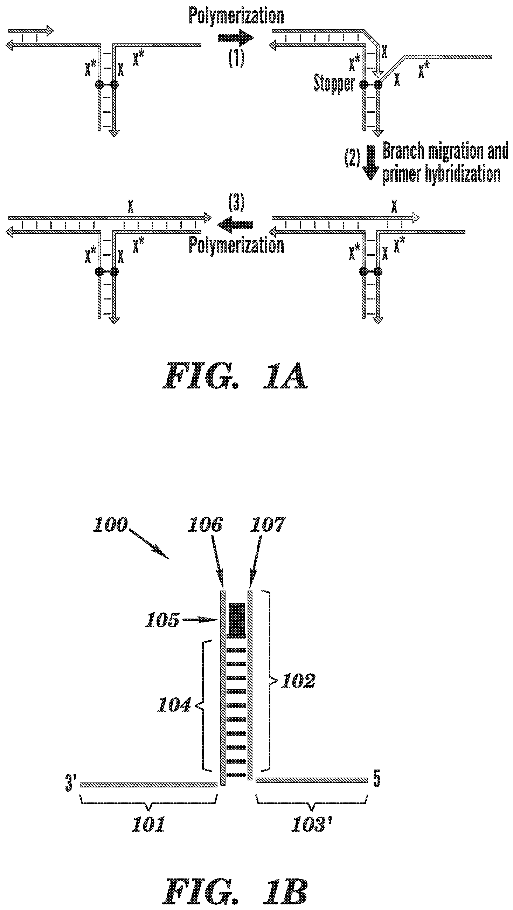

The current invention is based, in part, on the discovery that DNA polymerase displacement can be leveraged to synthesize any desired DNA sequence by using a specific nucleic acid template secondary structure (**FIG. 1A**). Generally, and without limitation, the nucleic acid template can comprise oligonucleotides hybridized together to form a junction or several junctions that stop the DNA polymerase from synthesizing a nucleic acid sequence straight across the guide strand with complementary nucleotides, but allows for the DNA polymerase to displace and cross over the junction. Polymerization can then continue on a second guide strand and copy along a new backbone.

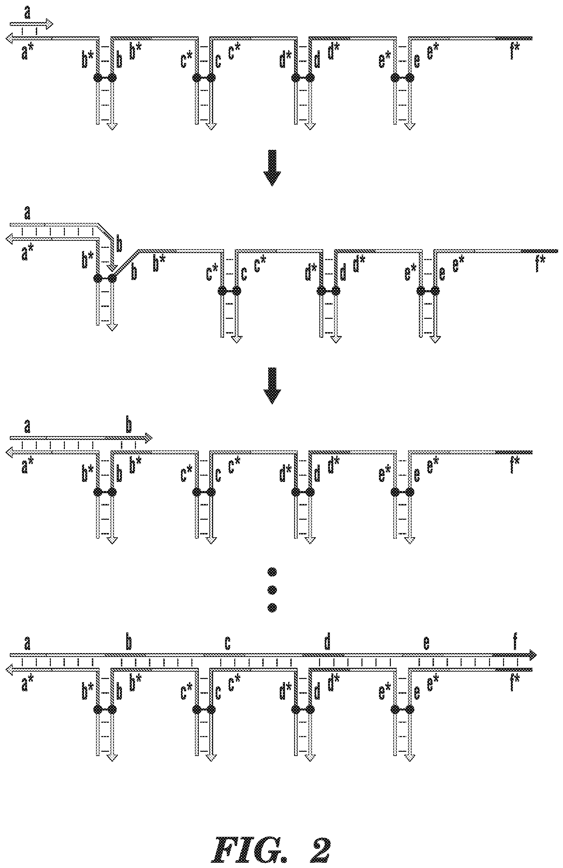

By hybridizing the multiple oligonucleotides together, called guide strands, the cross-junction synthesis can be cascaded to form longer sequences (**FIG. 2**). Multiple guide strands can be hybridized to make up a nucleic acid template of any length. The nucleic acid template can be used for synthesizing a nucleic acid sequence of any desired length.

Accordingly, in a first aspect the present invention is a nucleic acid template comprising: (a) a first guide strand comprising in 3' to 5' direction a first synthesis region, a second synthesis region, a first junction domain, and a first blocking region; and (b) a second guide strand comprising in 3' to 5' direction a second blocking region, a second junction domain and a third synthesis region, wherein the first junction domain comprises a nucleotide sequence substantially identical to a nucleotide sequence of the third synthesis region, and the first junction domain and second junction domain are substantially complementary to each other and form a double-stranded region, and wherein the first blocking region and the second blocking region together form a first blocking domain that blocks strand displacement activity of a polymerase. For example, a nucleic acid template may be for use in the synthesis of a nucleic acid sequence by a method described herein. The nucleic acid template comprises a region, *e.g.,* a double-stranded region, that can inhibit progression of a strand displacing polymerase. The double-stranded region along with the sequence at the 3'-end and the 5'-end can also be referred to as a synthesis domain or synthesis region herein.

As shown in **FIG. 1B****,** an exemplary cross-junction (**100**) comprises in 3' to 5' direction a synthesis region (**101**), a double-stranded region (**102**), and another synthesis region (**103**). The 3'-end (**104**) of the double-stranded region (**102**) has a sequence substantially similar to a nucleic acid sequence of the synthesis region (**103**) at the 5'-end of the duplex. The double-stranded region (**102**) further comprises a blocking domain (**105**) at the 5'-end of the region (**104**), wherein the blocking domain (**105**) is capable of blocking strand-displacement activity of a polymerase. The cross-junction can be one continuous polynucleotide or the cross-junction can be prepared by annealing two separate guide-strands (**106** and **107**).

The nucleic acid template can comprise any number of cross-junctions. For example, the nucleic acid template can comprise two or more, *e.g.,* three, four, five, six, seven, eight, nine, ten or more cross-junctions. When the nucleic acid template comprises two or more cross-junctions, they can be linked together via a nucleic acid linker. For example, a 5'-end of a first cross-junction can be linked to a 3'-end of a second cross-junction. Without limitations the linker can be of any desired length and/or nucleotide sequence. For example, the linker can simply be a nucleic acid backbone linkage *e.g*., phosphodiester linkage. In addition, the nucleic acid linkers can all be the same, all different, or some are the same and some are different.

Furthermore, when the nucleic acid template comprises two or more of the crossjunctions, the two or more of the cross-junctions can all be the same, some can be the same, some can be different, and/or all can be different. For example, the synthesis region (**101**) of two or more cross-junction can have a substantially identical nucleotide sequence; the synthesis region (**103**) of two or more cross-junctions can have a substantially identical nucleotide sequence; and/or the synthesis region (**101**) of a first cross-junction can have a substantially identical nucleotide sequence to the synthesis region (**103**) of a second cross-junction.

A cross-junction can be prepared from two separate nucleic acid strands, e.g., guide strands. Accordingly, the nucleic acid template comprises a first guide strand comprising in 3' to 5' direction a first synthesis region, a second synthesis region, a first junction domain, and a first blocking region; and a second guide strand comprising in 3' to 5' direction a second blocking region, a second junction domain and a third synthesis region, wherein the first junction domain comprises a nucleotide sequence substantially identical to a nucleotide sequence of the third synthesis region, and the first junction domain and second junction domain are substantially complementary to each other and form a double-stranded region, and wherein the first blocking region and the second blocking region together form a first blocking domain that blocks strand displacement activity of a polymerase. Without limitation, the above-noted regions and domains can independently comprise any nucleotide sequence provided that the nucleotide sequence of the first junction domain of the first guide strand and the third synthesis region of the second guide strand are substantially identical, and the nucleotide sequence of the first junction domain of the first guide strand and second junction domain of the second guide strand are substantially complementary to each other and form a double-stranded region.

In a second aspect, is a method for forming a nucleic acid template comprising annealing or hybridizing a first guide strand and a second guide strand, wherein (a) the first guide strand comprises in 3' to 5' direction a first synthesis region, a second synthesis region, a first junction domain, and a first blocking region; and (b) the second guide strand comprises in 3' to 5' direction a second blocking region, a second junction domain and a third synthesis region, wherein the first junction domain comprises a nucleotide sequence substantially identical to a nucleotide sequence of the third synthesis region, and the first junction domain and second junction domain are substantially complementary to each other and form a double-stranded region and wherein the first blocking region and the second blocking region together form a blocking domain that blocks strand displacement activity of a polymerase.

Disclosed herein is a reaction mixture comprising a nucleic acid template provided herein.

Disclosed herein is a reaction mixture that can be utilized to create the guide strands by addition of junction regions onto existing nucleic acid strands enzymatically or chemically. For example, a reaction mixture that can be utilized to create the guide strands by addition of blocking regions onto existing nucleic acid strands enzymatically or chemically.

Disclosed herein is a kit comprising the nucleic acid template provided herein.

Also disclosed herein is a kit comprising components or reaction mixture for creating the nucleic acid template described herein from nucleic acid sequences (partial guide strands) by addition of junction regions and/or blocking regions onto existing nucleic acid strands enzymatically or chemically.

In a third aspect is a method for synthesizing a nucleic acid sequence, the method comprising: (a) providing or obtaining a nucleic acid template of any one of claims 1-11; (b) annealing or hybridizing a primer, if not already annealed, to the nucleic acid template; and (c) extending a nucleotide sequence using a DNA polymerase having strand displacement activity from the 3'-terminus of the primer; optionally wherein the method further comprises amplifying the nucleic acid sequence or further comprises sequencing the synthesized nucleic acid sequence.

In a fourth aspect, is a method for synthesizing a nucleic acid sequence, the method comprising: (a) annealing or hybridizing a primer, if not already annealed, to a first guide strand, wherein the first guide strand comprises in a 3' to 5' direction a first synthesis region, a second synthesis region, a junction domain and a blocking region; and (b) extending a nucleotide sequence using a DNA polymerase having strand displacement activity from the 3'-terminus of the primer; (c) adding a second guide strand to the synthesis reaction, where the second guide strand comprises in a 3' to 5' direction a blocking region, a junction domain, a first synthesis region, a second synthesis region, a second junction domain, and a second blocking region, wherein the first blocking domain of the second guide strand and the blocking domain of the first guide strand together form a blocking domain that blocks strand displacement activity of a polymerase, the first junction domain of the second guide strand comprises a nucleotide sequence substantially complementary to a nucleic acid sequence of the junction domain of the first guide strand to form a double-stranded region, and the first synthesis region of the second guide strand comprises a nucleotide sequence substantially identical to a nucleic acid sequence of the junction domain of the first guide strand; (d) optionally, cross-linking the first blocking domain of the second guide strand and the blocking domain of the first guide strand; (e) further extending the nucleotide sequence; (f) optionally, adding additional new guide strands to the synthesis reaction and further extending the nucleotide sequence prior to addition of each additional new guide strand, wherein each additional guide strand comprises in a 3' to 5' direction a blocking region, a junction domain, a first synthesis region, a second synthesis region, a second junction domain, and a second blocking region, wherein the first blocking region of the new guide strand and the second blocking region of the last guide strand added to the synthesis reaction together form a blocking domain that blocks strand displacement activity of a polymerase; the first junction domain of the new guide strand comprises a nucleotide sequence substantially complementary to a nucleic acid sequence of the second junction domain of the last guide strand added to the synthesis reaction to form a double-stranded region; and the first synthesis region of the new guide strand comprises a nucleotide sequence substantially identical to a nucleic acid sequence of the second junction domain of the last guide strand added to the synthesis reaction; (g) optionally, crosslinking the first blocking region of the new guide strand and the second blocking region of the last guide strand added to the synthesis reaction prior to further extending the nucleotide sequence; optionally further comprising: (h) adding a terminal guide strand to synthesis reaction, where the terminal guide strand comprises in a 3' to 5' direction a blocking region, a junction domain, a first synthesis region, a second synthesis region, a second junction domain, and a second blocking region, wherein the first blocking region of the terminal guide strand and the second blocking region of the last guide strand added to the synthesis reaction together form a blocking domain that blocks strand displacement activity of a polymerase; the first junction domain of the terminal guide strand comprises a nucleotide sequence substantially complementary to a nucleic acid sequence of the second junction domain of the last guide strand added to the synthesis reaction to form a double-stranded region; and the first synthesis region of the terminal guide strand comprises a nucleotide sequence substantially identical to a nucleic acid sequence of the second junction domain of the last guide strand added to the synthesis reaction; (i) optionally, cross-linking the first blocking domain of the terminal guide strand and the blocking domain of the last guide strand added to the reaction; and (j) further extending the nucleotide sequence; optionally wherein the method further comprises amplifying the nucleic acid sequence or further comprises sequencing the synthesized nucleic acid sequence. Additional guide strands may be added to the synthesis reaction, where the nucleotide sequence is extended prior to adding each additional guide strand.

In a fifth aspect, of the invention is the use of a nucleic acid template of the invention for: (a) creating combinatorial barcodes to assign unique identities to a target population, wherein the target population is surface positions/partitions, matrices, biomolecules, molecular libraries or biological material, optionally wherein the biological material is vesicles, cells, tissues, organoids, droplets, liposomes, small molecules, or beads, and optionally the use comprising splitting the target population prior to addition of a guide strand and pooling the split target population after addition of the guide strand; (b) creating combinatorial barcodes to assign spatial position identifiers to a target, wherein the target is a surface, biomolecule, or biological material, optionally wherein the biological material is biomolecules, cells, tissues, organs, organoids, vesicles, liposomes, or droplets; (c) determining the proximity of biomolecules and creating records indicative of distance information between labeled biomolecules; or (d) generating a nucleic acid sequence library.

### BRIEF DESCRIPTION OF THE DRAWINGS

**FIG. 1A** is a schematic representation of an exemplary cross-junction synthesis mechanism. First, a strand displacing polymerase copies along a template strand until reaching a stopper (step 1). The first template strand (left) is designed to have an **x*** domain, as shown, so that the complementary **x** sequence is appended on the 3' end of the growing strand. A second template strand (right) paired to the first template strand is designed to have an exposed **x*** binding domain and an **x** domain bound to the **x*** domain on the first template strand. When the **x** domain on the second template strand displaces the newly synthesized **x** domain on the 3' end of the growing strand through branch migration (Lee *et al.,* 1970), that strand is then free to pair this domain with the complementary exposed **x*** domain on the second template strand. At this point, the strand has now successfully `hopped' the junction to hybridize with the backbone of a new strand, and synthesis may continue.
**FIG. 1B** is a schematic representation of an exemplary cross-junction.
**FIG. 2** is schematic representation of an exemplary cascading cross-junction synthesis. A multitude of template strands may be hybridized to enable cascaded cross-junction synthesis reactions. Template strands with corresponding motif domains can be hybridized together, and a strand displacing polymerase can be used to copy across each junction. In the example above, a primer with domain **a** copies bases ending in domain **b** until it reaches the first stopper, where it can be then displaced and reach across the junction to the **b*** on the second template strand (from the left). Sequences up to **c, d,** and **e** are copied similarly (with optional additional bases in the grey domains intercalated). This method is able to concatenate arbitrary prescribed sequences, and only requires the fundamental domain constraints depicted in **FIG. 1****.**

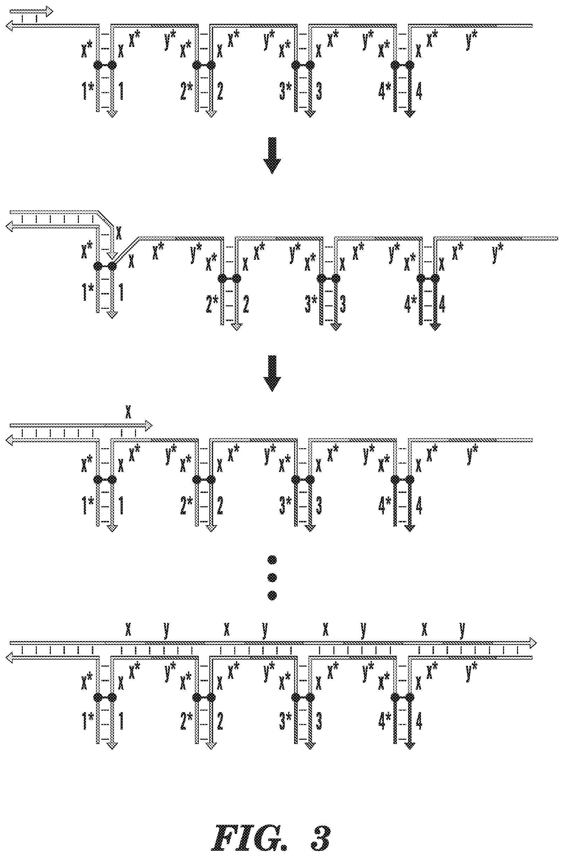

**FIG. 3** is a schematic representation showing synthesis of a fixed number of repeated sequences. By programming unique hybridization domains (**1, 2, 3,** and **4**) for each of the template strands, a fixed-length template concatemer can be formed for cross-junction synthesis. However, each of these template strands can be used for appending the same sequence as a previous junction (*e.g*. the **x y** sequence) if desired. This allows a fixed number of repeated sequence domains to be synthesized. For example, as shown in **FIG. 3****,** the sequence **x y** is copied into a sequence exactly 4 times.

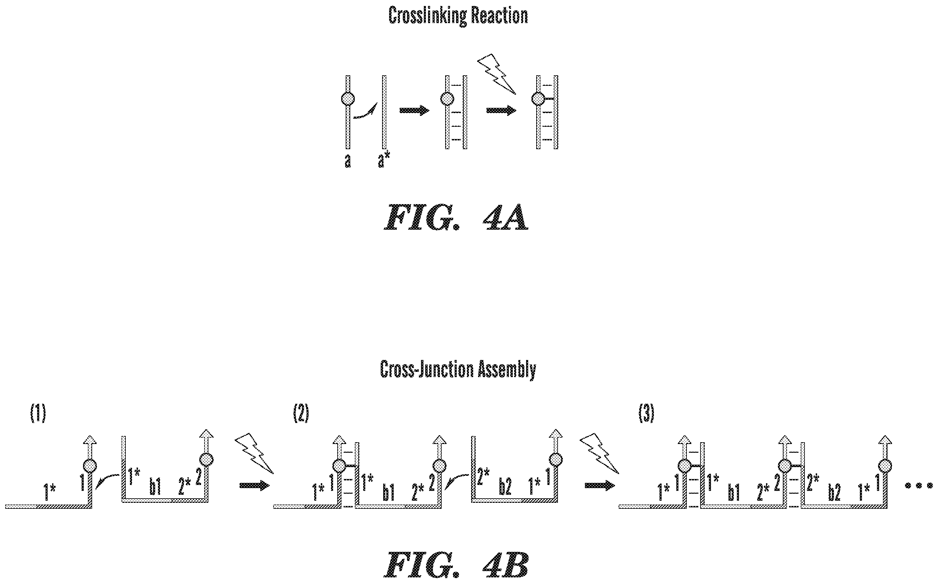

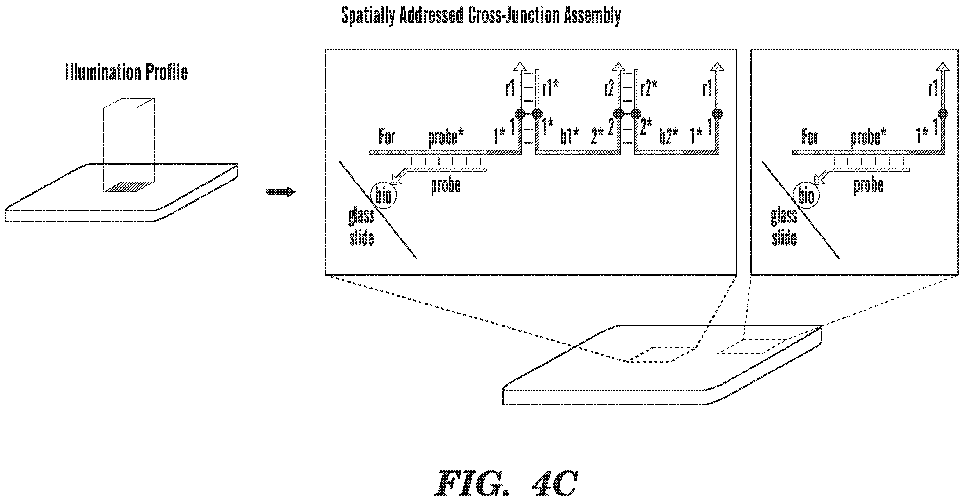

**FIGs. 4A-4C** are schematic overview of light-directed cross junction (blocking domain) assembly. **FIG. 4A** shows interstrand crosslinking reaction between complementary sequences can be achieved with a crosslinking base modification (filled cyan circle). (**FIG. 4B** shows iterative assembly of cross-junctions: (1) An initial docking strand with a crosslinking base modification and hybridization domain **1** is hybridized to a strand with the complement site **1*,** the first barcode sequence **b1,** the next hybridization domain **2** and a crosslinker base. (2) A UV crosslinking light source is used to covalently link the two sequences together, un-crosslinked sequences are washed away and the next iteration of strands carrying the **b2** sequence are introduced. (3) UV crosslinking and continuation of a growing sequence chain on a glass surface. **FIG. 4C** shows that photocrosslinked crossjunctions will only be created within the illuminated areas.

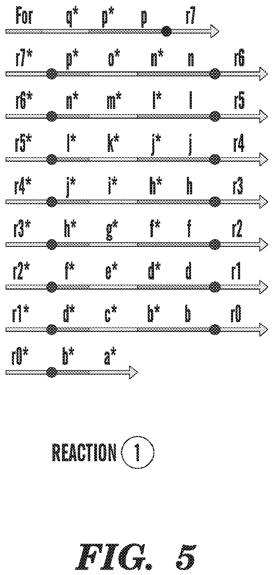

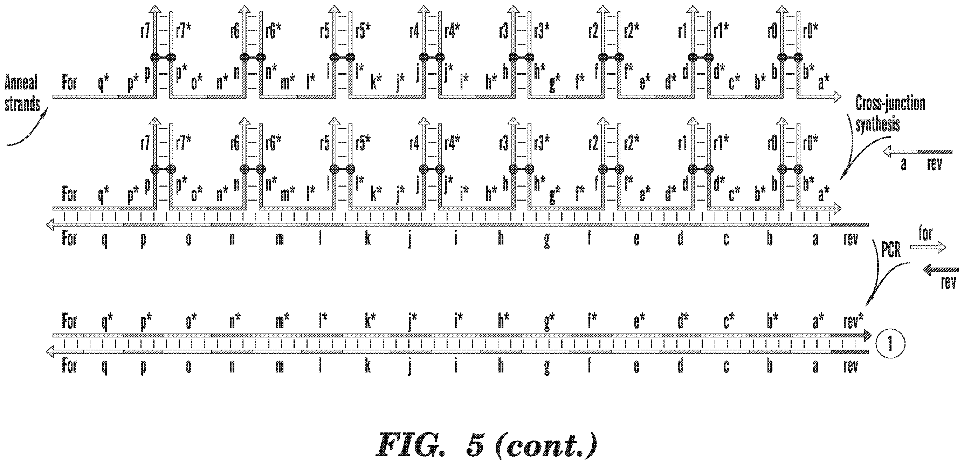

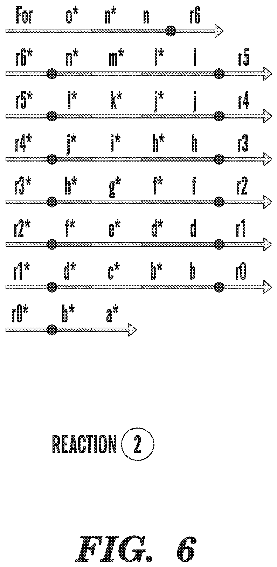

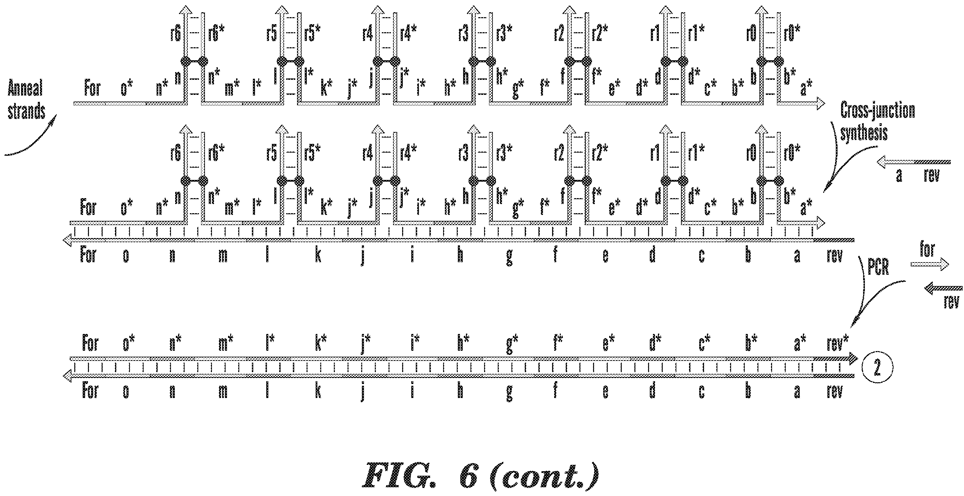

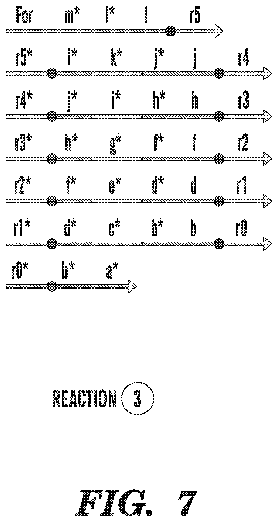

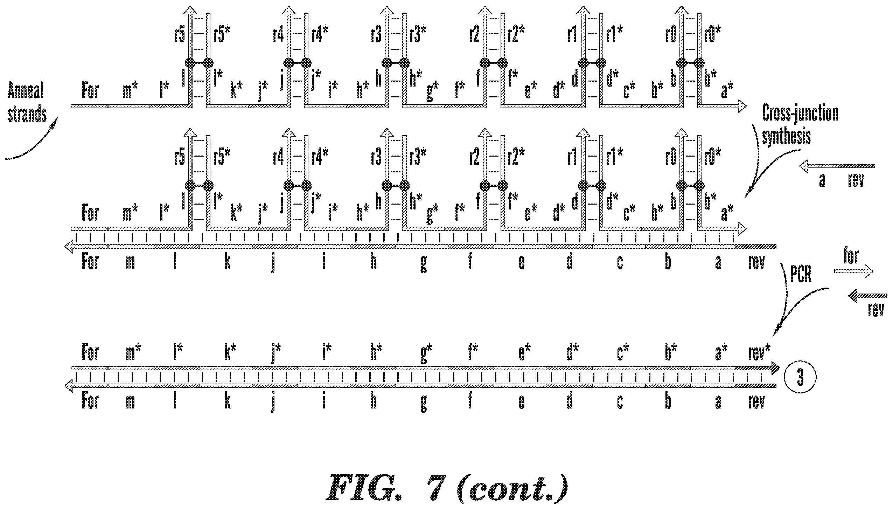

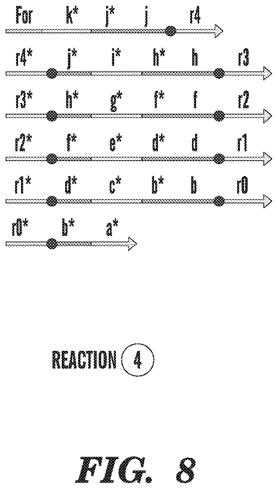

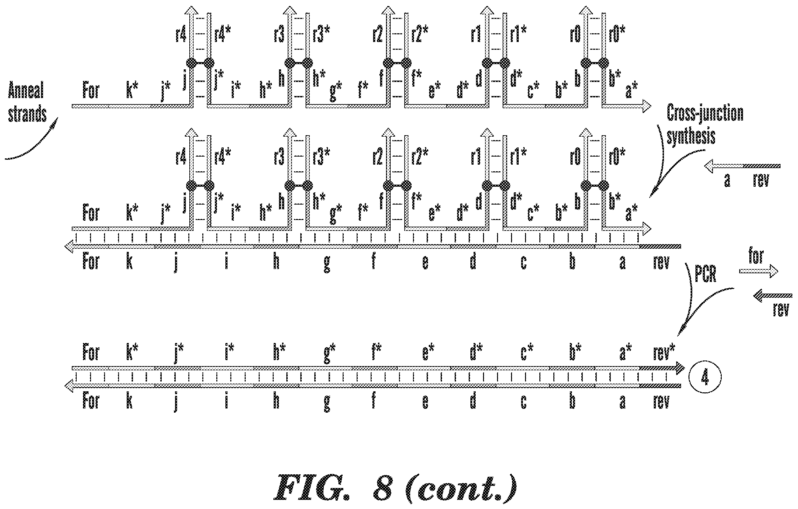

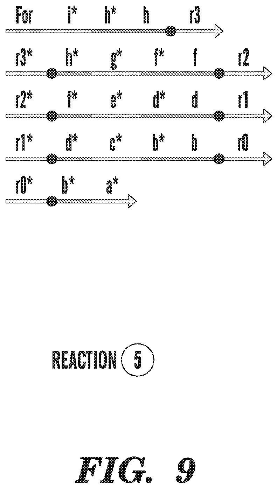

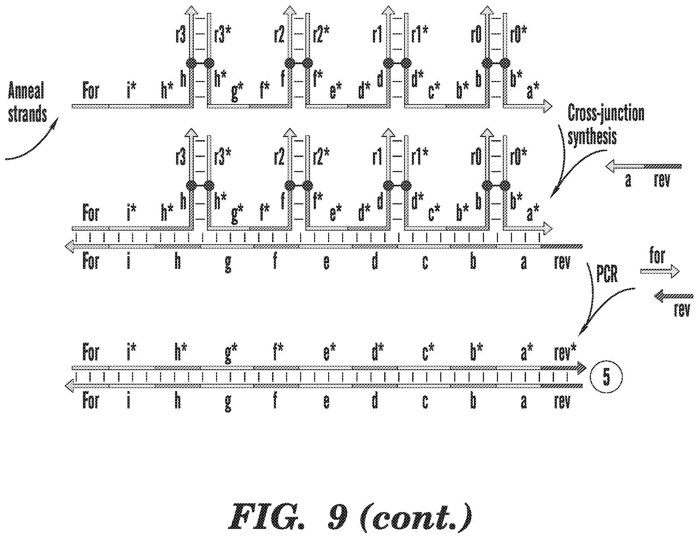

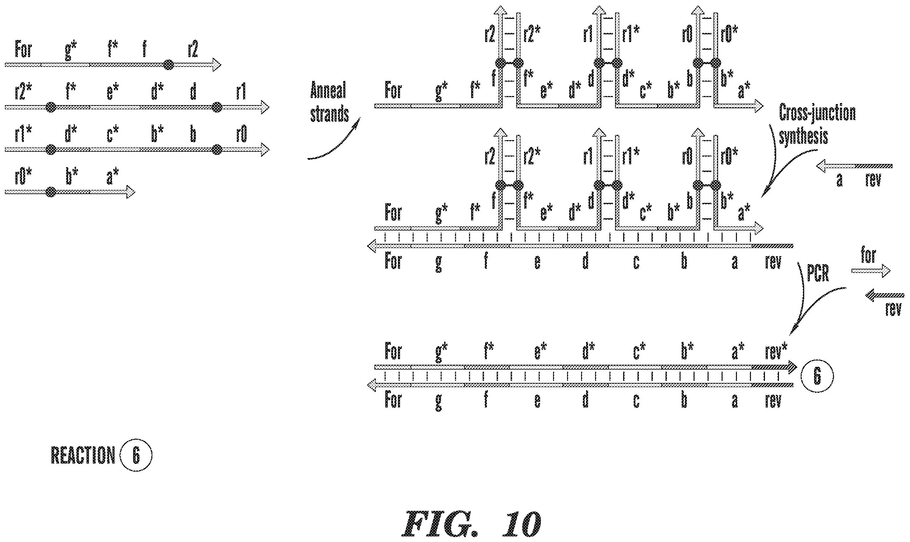

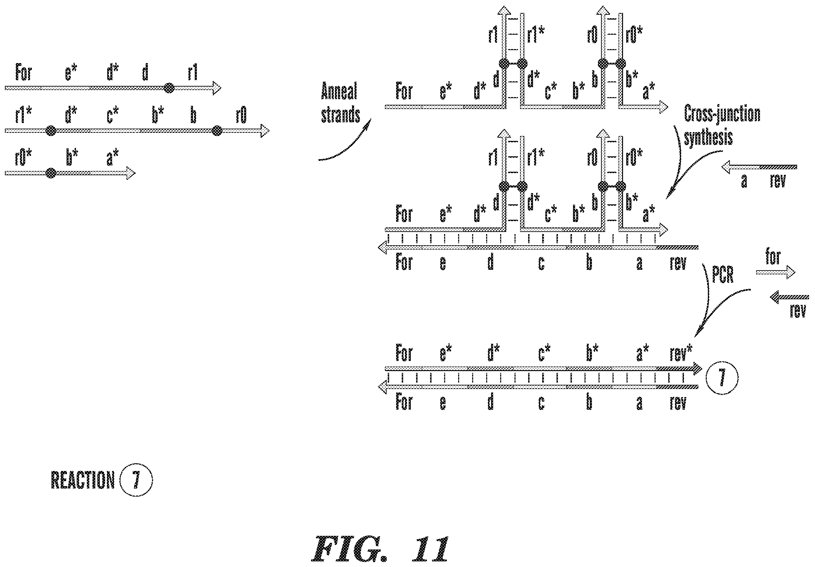

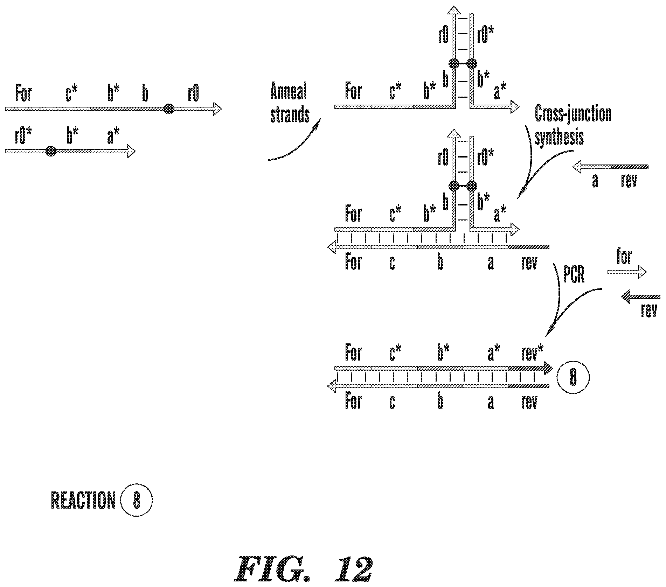

**FIGs. 5-12** are reaction schemes for synthesis of template nucleic acids and synthesis of a nucleic acid sequence therefrom for a template with 8-junctions or blocking domains (**FIG. 5**), 7-junctions (**FIG. 6**), 6-junctions (**FIG. 7**), 5-junctions (**FIG. 8**), 4-junctions (**FIG. 9**), 3-junctions (**FIG. 10**), 2-junctions (**FIG. 11**) and 1-junction (**FIG. 12**). First, template strands were annealed together. Then, a cross-junction synthesis reaction was performed with a primer as depicted. Finally, PCR was performed to amplify the synthesis product into a double-stranded amplicon.

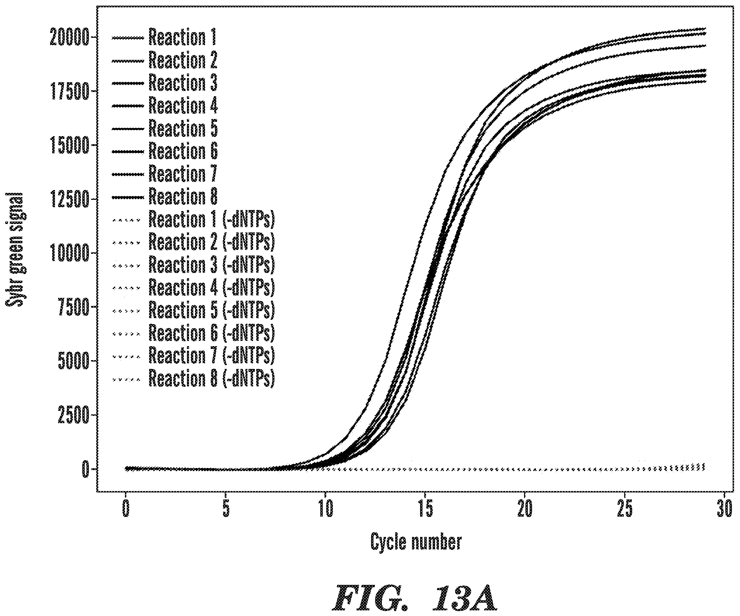

**FIG. 13A** shows Real-time qPCR amplification of the synthesis depicted in **FIGs. 5-12****.** PCR reactions corresponding to reactions 1-8 presented above (**FIGS. 5-12**) were monitored on a real-time PCR machine using an intercalating dye. All 8 cross-junction synthesis reactions were diluted 1000x (final) into PCR solutions and showed amplification around the same cycle. As a control, 8 reactions with all components except dNTPs were also mixed with the PCR master mix to check that amplicons did not result from the PCR step and template strands alone.

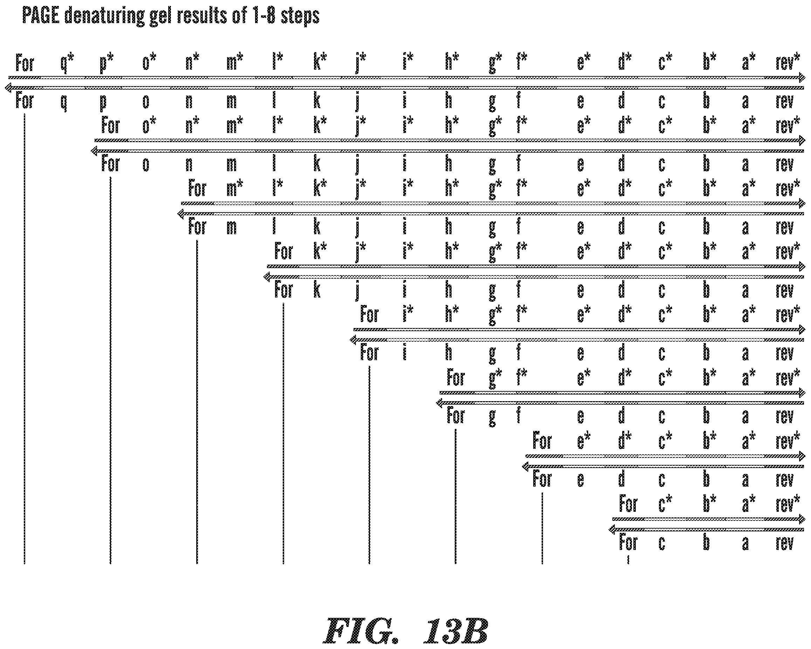

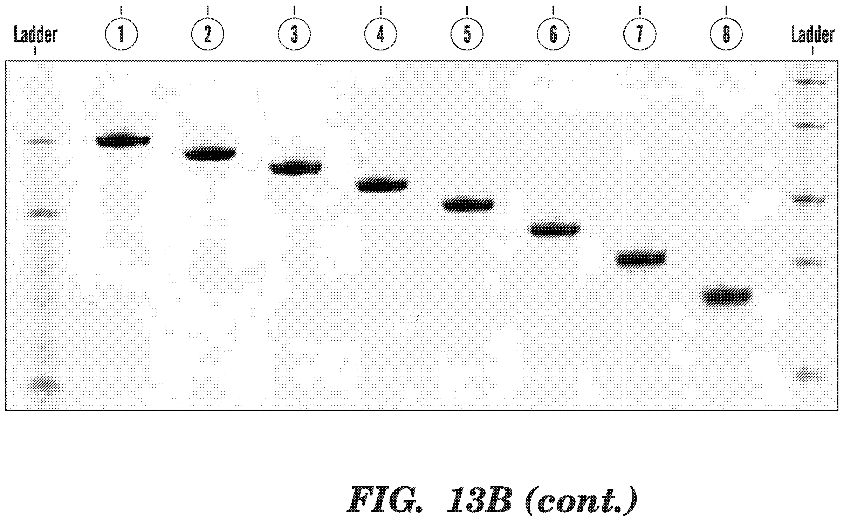

**FIG. 13B** is a PAGE denaturing gel showing the 8 synthesis products of expected length.

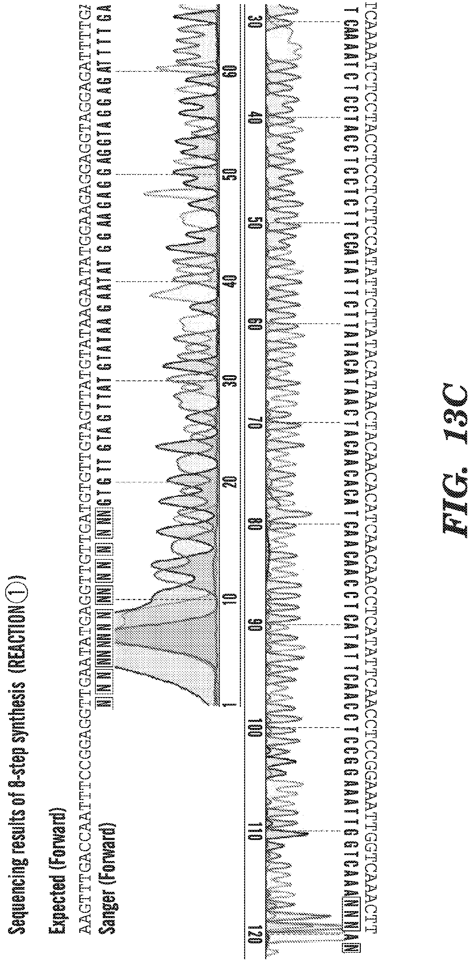

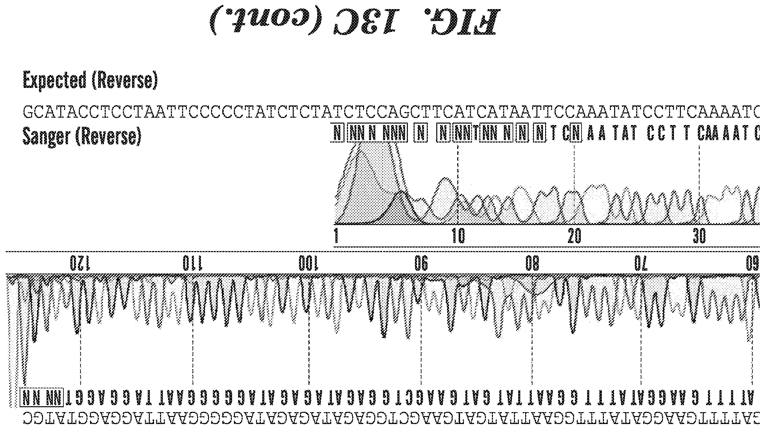

**FIG. 13C** shows Sanger sequencing results for the synthesis from the 8-junction template (**FIG. 5**) matched the programmed sequence in both directions.

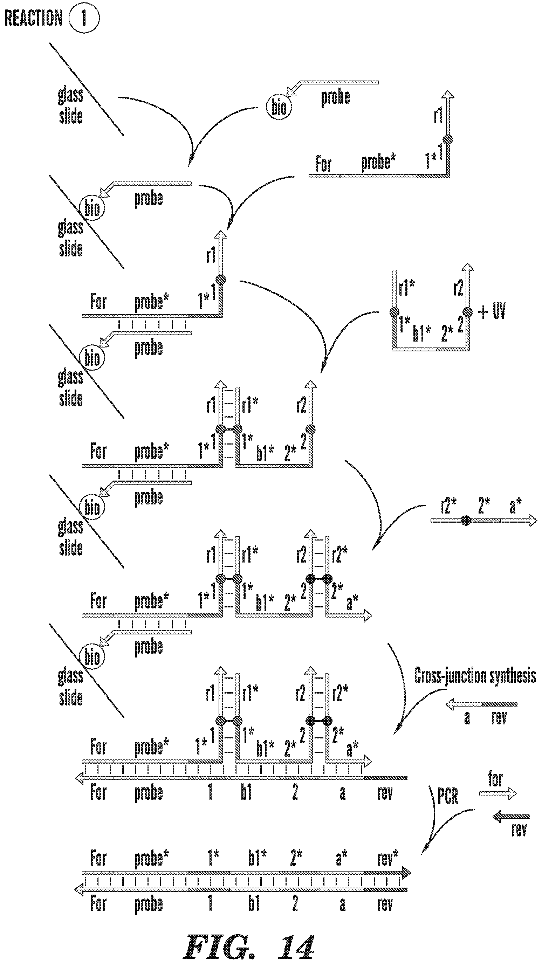

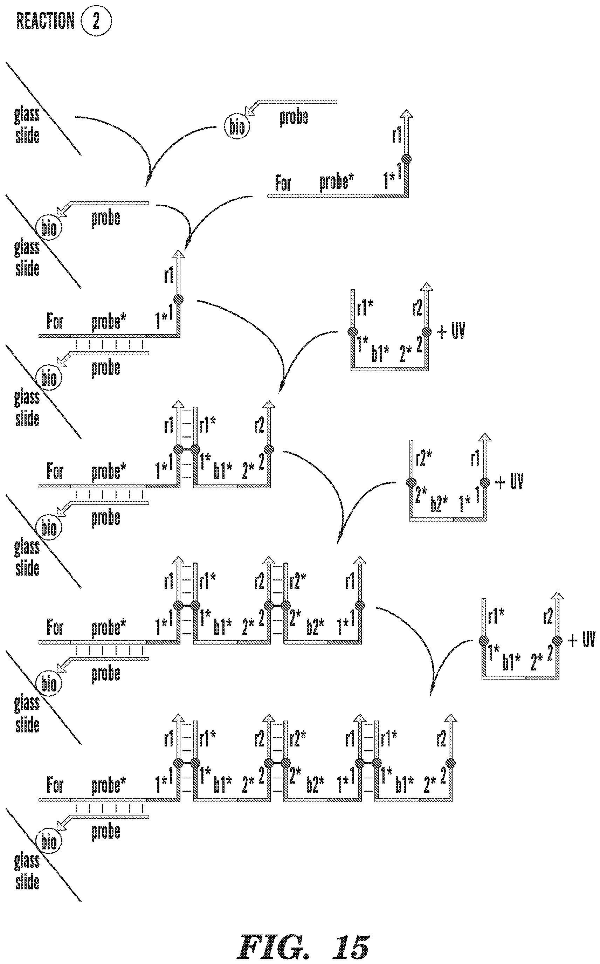

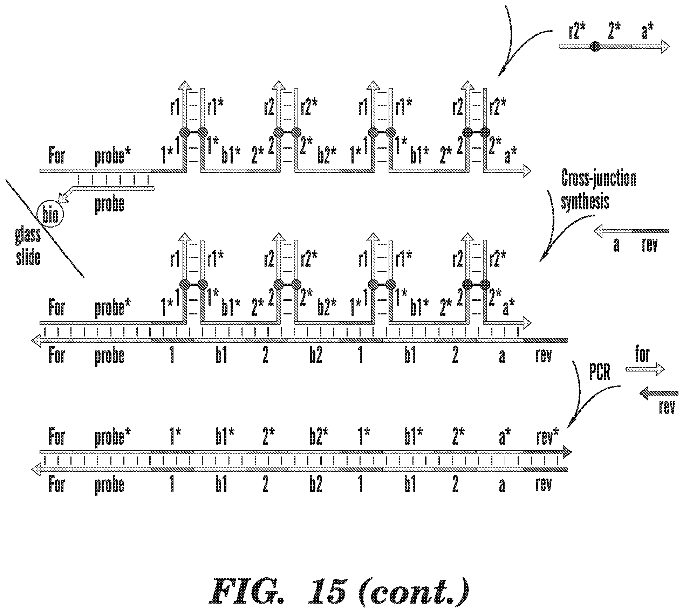

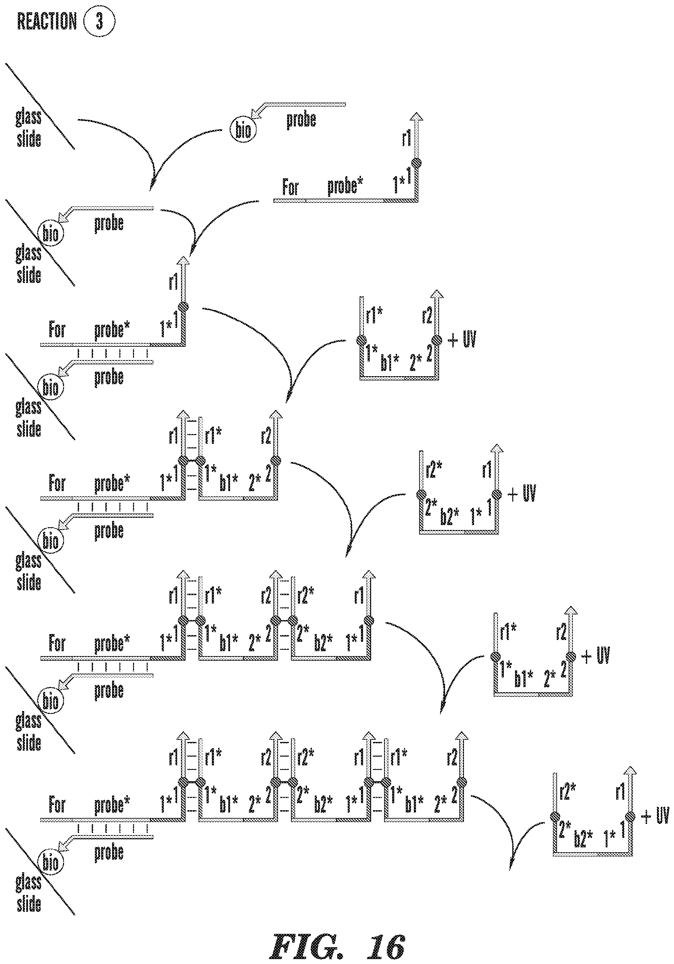

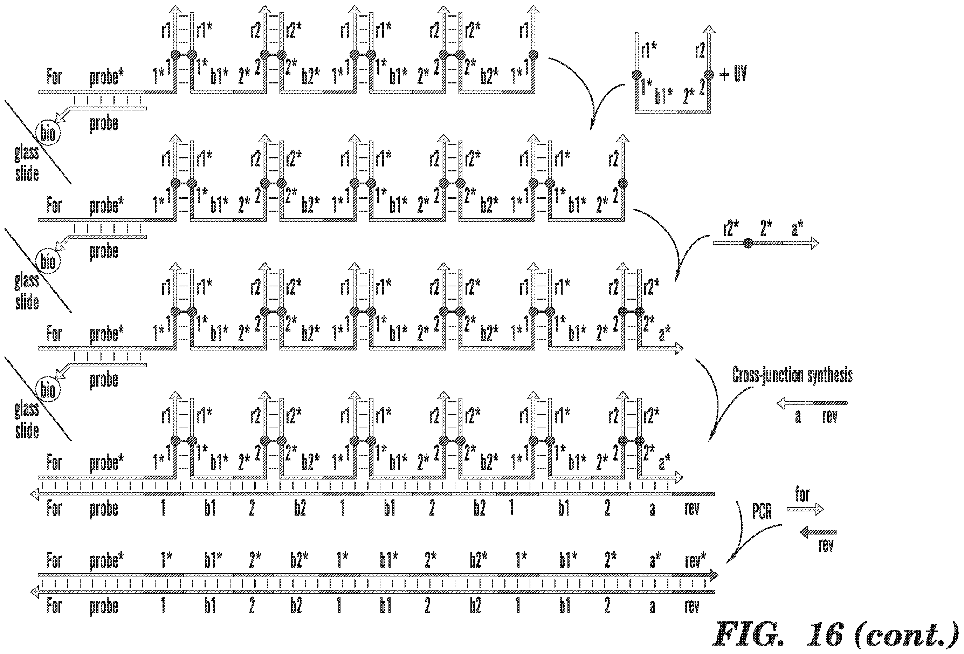

**FIGs. 14-16** are reaction schemes for synthesis of template nucleic acids by light-directed 2-junction (**FIG. 14**), 4-junction (**FIG. 15**) and 6-junction (**FIG 16**) concatemer formation followed by synthesis. A biotinylated probe sequence was attached to a glass slide through a biotin-streptavidin interaction. Template strands were introduced iteratively to form a template containing three strands, and then cross-junction synthesis was performed with the **rev a** primer. After dilution and PCR, the double-stranded product was formed.

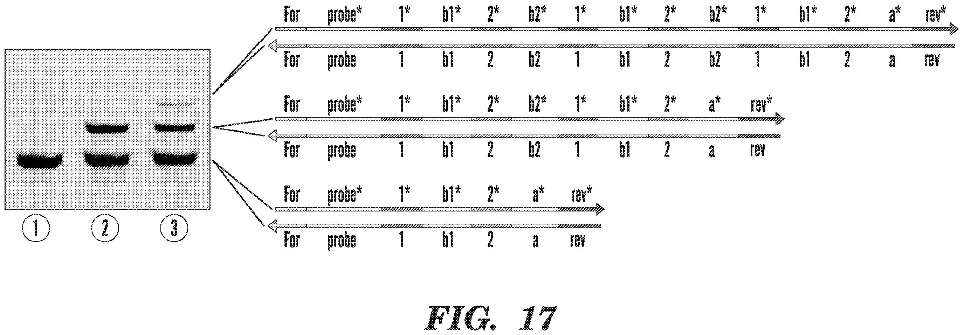

**FIG. 17** is a PAGE denaturing gel showing the readout of PCR products of the synthesis depicted in **FIGs. 5-12****.**

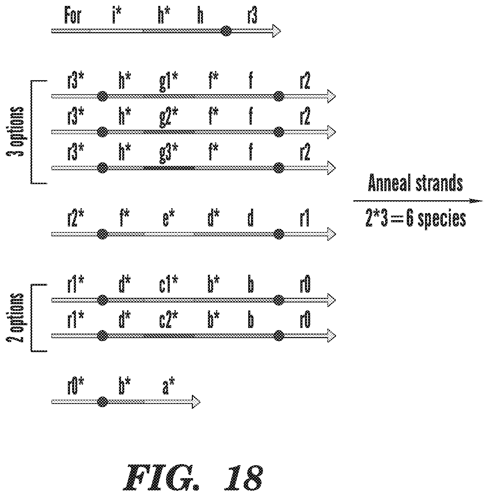

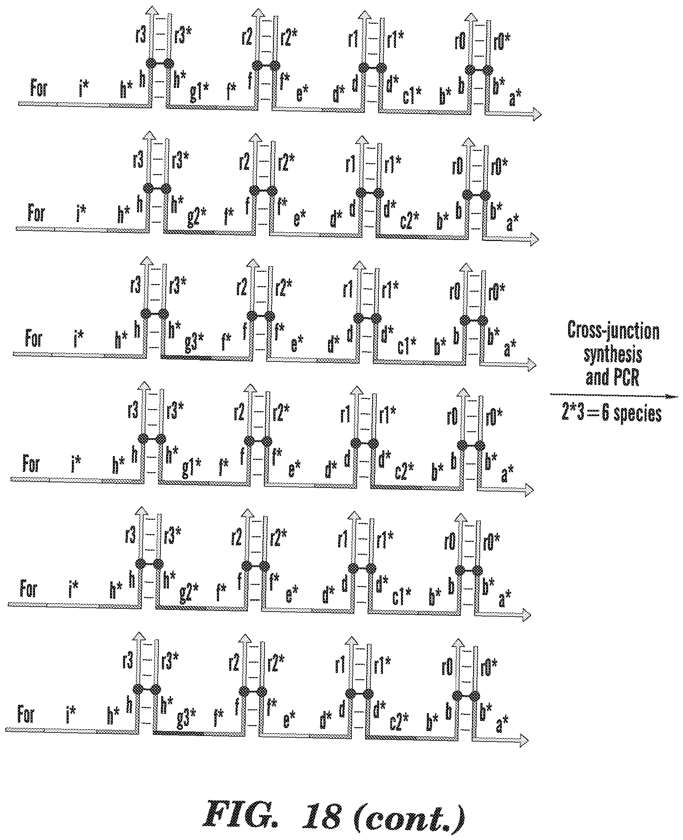

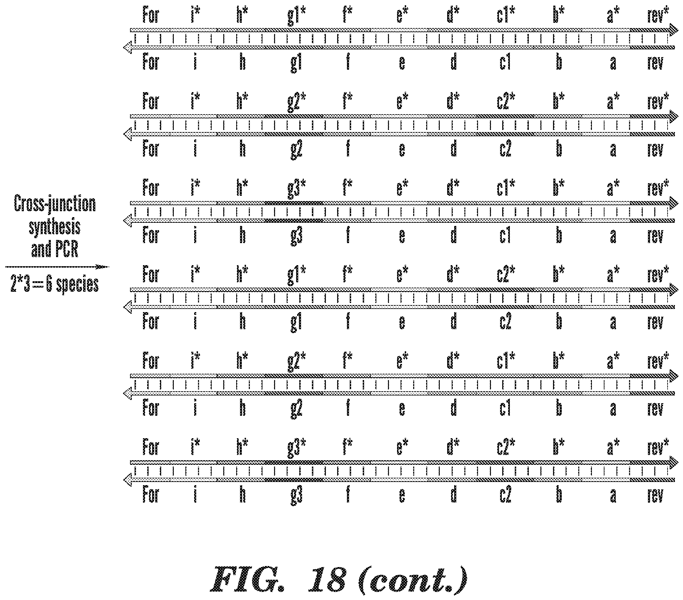

**FIG. 18** is a schematic representation of an exemplary combinatorial cross-junction synthesis. The example above shows a combinatorial library formed by the inclusion of three possible options for the second-from-left template strand and two options for the second-from-right template strand. By including all possibilities in the annealing reaction, a library of concatemers is formed, from which a library of synthesized sequences can be formed.

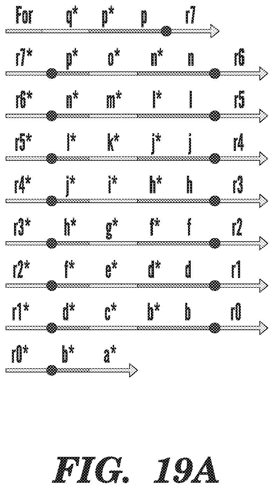

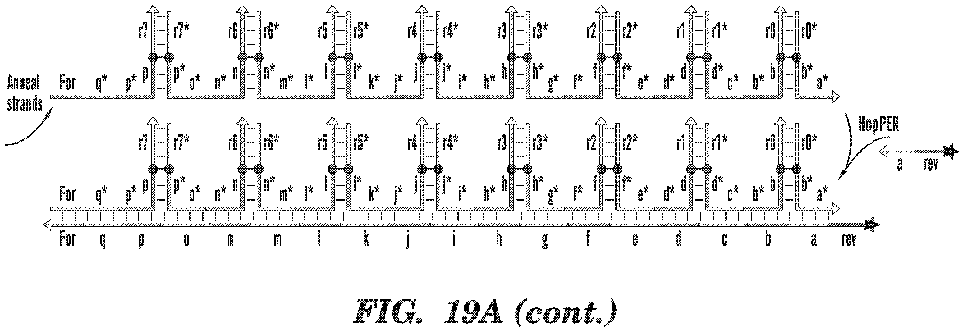

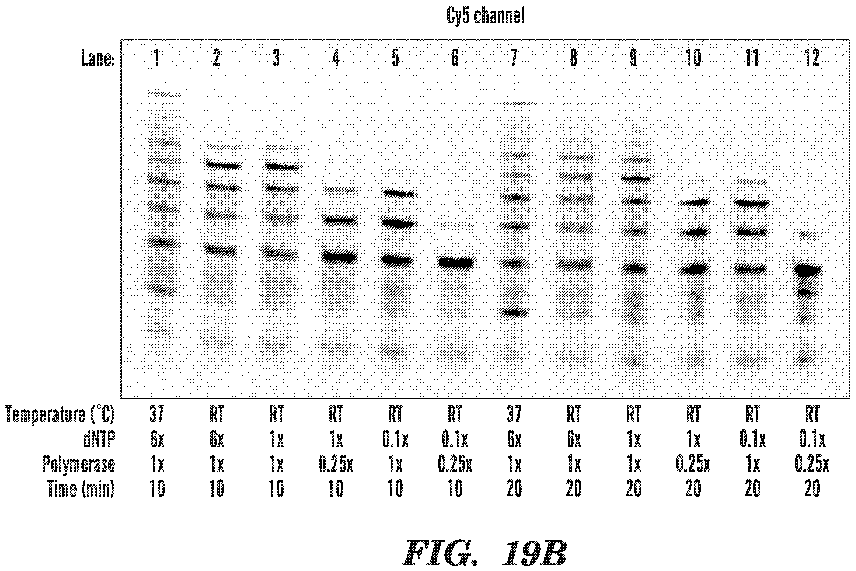

**FIG. 19A** is a schematic representation of synthesis of a template nucleic acid and synthesis of a nucleic acid strand therefrom where the full length template is assembled and added to the synthesis reaction. **FIG. 19B** shows results of synthesis scheme shown in **FIG. 19A** under different conditions as specified under each lane. Each band represents the extension steps. It can be seen that lanes 1 and 7 have highest efficiency.

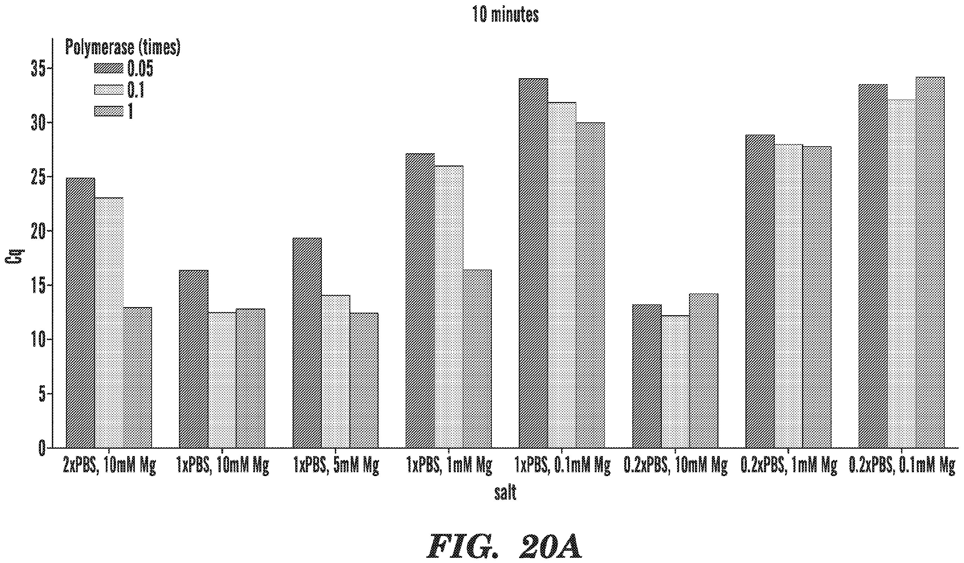

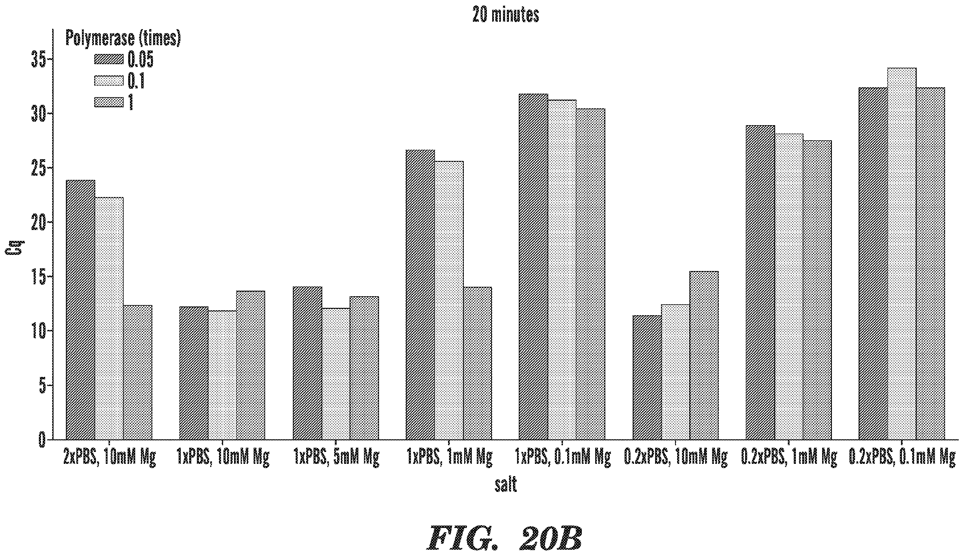

**FIG. 20A-20B** are bar graphs showing Cq values of qPCR reactions amplifying products of synthesis across 5-junctions performed under different salt and polymerase conditions with 10 minute (**FIG. 20A**) or 20 (**FIG. 20B**) minute incubations.

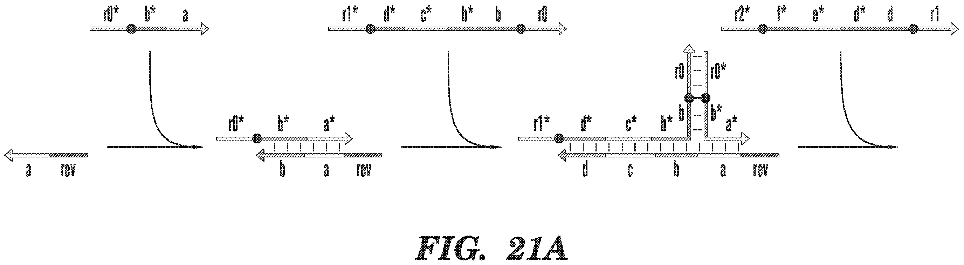

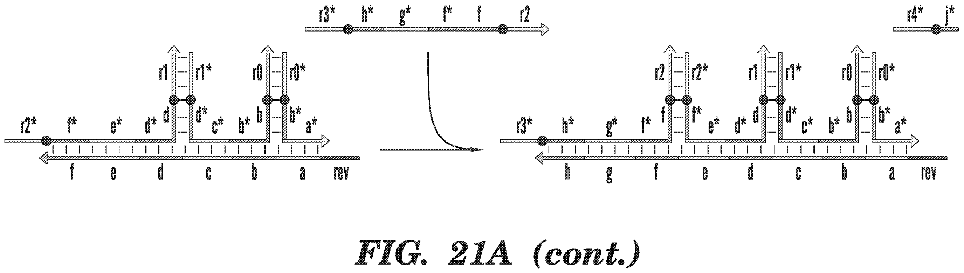

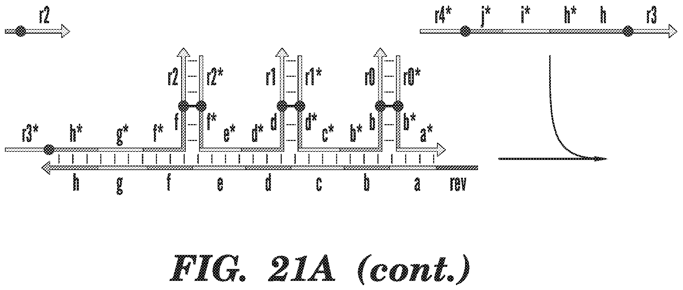

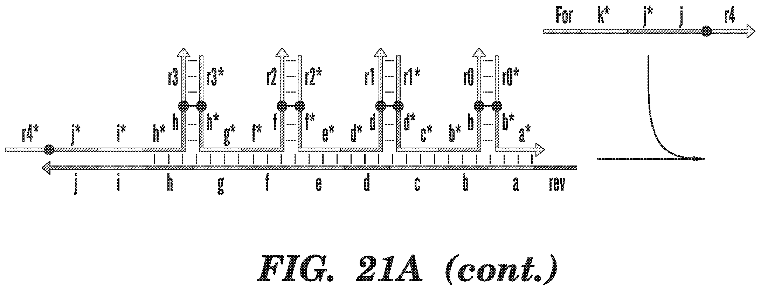

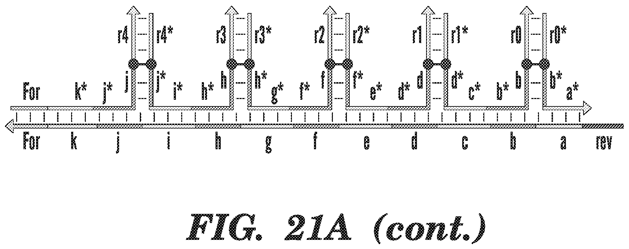

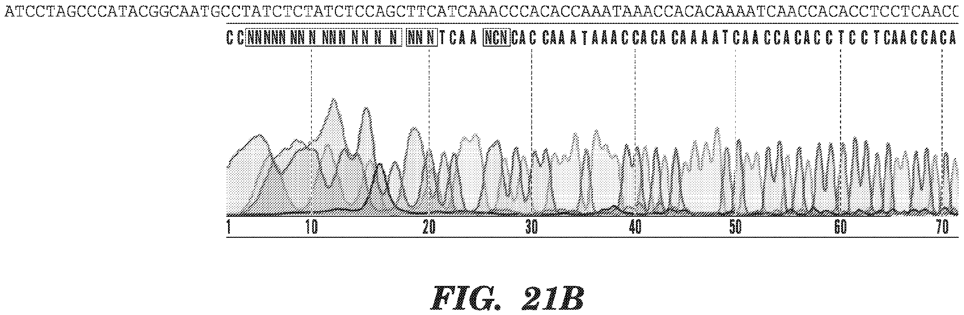

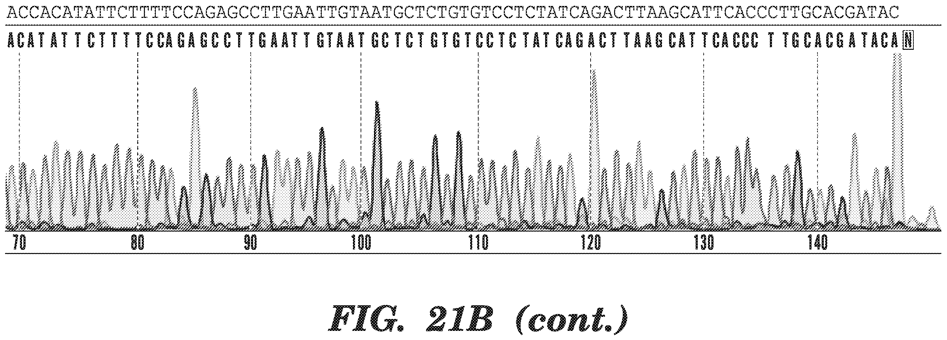

**FIG. 21A** shows a schematic representation of an exemplary nucleic acid synthesis method where guide strands are added consecutively to the reaction mixture during synthesis to assemble the full length template nucleic acid strand. **FIG. 21B** shows expected sequencing results (top) compared to Sanger sequencing results of PCR amplified product of synthesis across 5-junctions performed using an exemplary method depicted in **FIG. 21A****.**

### DETAILED DESCRIPTION

The fundamental strategy for cross-junction synthesis is depicted in **FIG. 1****.** Shown are two guide strands (also referred to as template strands) that have been hybridized together to form a junction, and a primer that has bound in front of that junction on the first template strand (left). A strand displacing polymerase is used to copy the **x** domain (also referred to as a junction domain herein) until it reaches a stopper (shown in black and also referred to as a blocking domain herein). Afterwards, the new and old **x** domains compete in a random walk branch migration process (Lee *et al.,* 1970). Ultimately, the new **x** domain can bind to the exposed **x*** domain on the second template strand (right, also referred to as a synthesis region herein), thus successfully crossing the junction. Polymerization can then continue on the second template strand, copying along a new backbone.

As used herein, the "nucleic acid template" or a "template nucleic acid" refers to a nucleic acid comprising at least two guide strands (**106** and **107**) forming at least one cross-junction (**100**). Without limitations, the nucleic acid template can comprise any number of cross-junctions (**100**), *i.e.,* the nucleic acid template can comprise any number for guide strands for forming the desired number of cross-junctions. Multiple guide strands can be hybridized to make up a full nucleic acid template that promotes synthesis of a desired nucleic acid sequence. **FIGs. 1A-2** provide guidance for the structure of an exemplary nucleic acid template based on secondary structure and hybridization of the first and second guide strand. The nucleic acid template can be designed based on the desired nucleotide sequence to be synthesized.

The nucleic acid template comprises one or more cross-junctions. Generally, each cross-junction comprises in a 3' to 5' direction a first guide strand and a second guide strand. The first guide strand comprises in a 3' to 5' direction optionally a second junction domain and a second blocking region. The second guide strand comprises in a 3' to 5' direction optionally a third junction domain and optionally a third blocking region.

When the nucleic acid template comprises two or more cross-junctions, the first guide strand of a first cross-junction can be a second guide strand of a second cross-junction. Likewise, the second guide strand of a first cross-junction can be a first guide strand of a second cross-junction.

It is noted that a guide strand described herein can comprise a barcode domain. For example, the second guide strand of a junction can comprise barcode domain at the 5'-terminus. One of the first or second synthesis regions of a guide strand may comprise a barcode domain.

As used herein, a "barcode domain," refers to the part of a strand, *e.g.,* a guide strand that comprises a nucleic acid sequence that represents information or data. Non-limiting examples include spatial information, sequences, molecule sequences, experiment/batch number, binary codes, random barcodes to create unique molecular identifiers (UMI's), or any combination thereof. The barcode domain sequence can be predetermined by a barcode library. The barcode domain can be any suitable sequence. A barcode domain can be assigned a bit value. For example, each barcode domain can be independently assigned a bit value. It is noted that bit values are not limited to 0 and 1. The barcode domain sequence can be 0 nucleotides in length. For example, lack of a barcode sequence can provide information (*e.g*, a 0 bit value is assigned to the absence of a barcode and a 1 bit value can be represented by a barcode domain of 1 or more nucleotides in length).

The barcode domain may be immobilized on a substrate surface. The barcode may be immobilized in a predetermined pattern. The barcode domain may represent spatial information.

A guide strand described herein can also comprise a primer sequence. For example, the first guide strand of a junction can comprise a primer sequence at the 3'-terminus. In another non-limiting example, the second guide strand of a junction can comprise a primer sequence at the 5'-terminus.

In some embodiments, the second guide strand further comprises a fourth synthesis region at the 5' end of the third synthesis region; optionally wherein the second guide strand further comprises at its 5' end in 3' to 5' direction a third junction domain and a third blocking region, and the nucleic acid template further comprises a third guide strand comprising in 3' to 5' direction a fourth blocking region, a fourth junction domain and a fifth synthesis region, wherein the third junction domain comprises a nucleotide sequence substantially identical to a nucleotide sequence of the fifth synthesis region, wherein the third junction domain and fourth junction domain are substantially complementary to each other and form a double-stranded region, and wherein the third blocking region and the fourth blocking region together form a second blocking domain that blocks strand displacement activity of a polymerase.

As used herein, the terms "guide strand" or "template strand" are used interchangeably to refer to part of the nucleic acid template and serves as the basic building block of the nucleic acid template. Several guide strands can be combined to make up the nucleic acid template used for synthesizing a desired output nucleic acid sequence. The first guide strand comprises in the 3' to 5' direction a first synthesis region, a second synthesis region, a first junction domain, and a first blocking region. The second guide strand also comprise in 3' to 5' direction a second blocking region, a second junction domain and a third synthesis region. For reference, several exemplary guide strands are depicted in **FIG. 2****.** A set of guide strands (template strands) can be used to assemble the full length template nucleic acid used for nucleic acid synthesis.

As used herein, a "synthesis region" refers to part of a template strand (guide strand) that is to be copied to the nucleic acid sequence being synthesized. Without limitation, each synthesis region can independently comprise any desired nucleotide sequence. In other words, each synthesis region can be independently of any length. For example, each synthesis region can be one-nucleotide to few thousand nucleotides in length. Each synthesis region may be 1,000-nucleotides or less, 750-nucleotides or less, 500-nucleotides or less, 400-nucleotides or less, 300-nucleotides or less, 250-nucleotides or less, 200-nucleotides or less, 150-nucleotides or less, 100-nucleotides or less in length or 50-nucleotides or less in length. Without limitations, a synthesis region can be just one nucleotide.

Without limitation, the double-stranded region of the cross-junction can independently comprise any desired nucleotide sequence or number of base-pairs. In other words, the double-stranded region can be independently of any length. For example, each double-stranded region can be one base pair to tens of base pairs in length. The double-stranded region can be independently at least one, at least two, at least three, at least four, at least five, at least six, at least seven, at least eight, at least nine or at least ten nucleotides or base pairs in length.

As used herein, a "junction domain" refers to part of a guide strand that can hybridize with part of a second guide strand to form a duplex, and a nucleic acid sequence produced from a nucleic acid template comprising the guide strand comprises: (i) a sequence complimentary to the nucleotide sequence of the junction domain; or (ii) a sequence complimentary to the reverse complement of the nucleotide sequence of the junction domain. In other words, a nucleic acid sequence produced from a nucleic acid template comprising the guide strand comprises either a sequence synthesized from the junction domain sequence or the reverse complement of the junction domain sequence.

Without limitation, each junction domain can independently comprise any desired nucleotide sequence or number of nucleotides. In other words, each junction domain can be independently of any length. For example, each junction domain can be one nucleotide to tens of nucleotides in length. Each junction domain can be independently one, two, three, four, five, six, seven, eight, nine or ten nucleotides in length.

As noted above, junction domain of a first guide strand can hybridize with a junction domain of a second domain to form a double-stranded structure. Without limitations, each duplex region can independently comprise any desired number of base-pairs. In other words, each duplex region can be independently of any length. For example, each duplex region can be one base pair to tens of base pairs in length. Each duplex region can be independently one, two, three, four, five, six, seven, eight, nine or ten nucleotides or base pairs in length.

As used herein, a "blocking domain" refers to part of the nucleic acid template that can prevent or stop a polymerase from continuing moving along the backbone of a template strand and/or copying the template strand. For example, the blocking domain can be a double-stranded region having high stability, e.g., a super-stable double-stranded region such that a polymerase is unable to separate the two strands. Methods for forming super-stable double-stranded nucleic acids are well known in the art and amenable to the invention. For example, the blocking domain can comprise one or more modified nucleotides known in the art for increasing *Tₘ* of double-stranded nucleic acids. Exemplary such nucleotides include, but are not limited to locked nucleic acids (LNAs), 2'-O-methoxy-ethyl (2'-MOE) nucleotides, 2,6-diamopurine, G-clamp (an analog of C having 4 hydrogen bonds) and guanidinium G-clamp nucleotides, and the like.

In some embodiments, the blocking domain can comprise covalent cross-linking of two guide strands. It is noted that covalent cross-linking can comprise the situation where the nucleotide at the 5'-terminus of the first guide strand is cross-linked to the nucleotide at 3'-end of the second template strand, *e.g*., via an oligonucleotide or a single nucleic acid backbone linkage (*e.g*., phosphodiester bond). Generally, covalent cross-linking of two guide strands excludes which strand can lead to one continuous sequence from the two guide strands. Thus, at least one (or both) of the nucleotides involved in the covalent cross-linkage may not be a terminus nucleotide. In other words, at least one (or both) of the nucleotides involved in the cross-linking may not be the first or last nucleotide of the template strand.

Without limitation, each blocking domain can independently comprise any desired nucleotide sequence or number of base-pairs. In other words, each blocking region can be independently of any length. For example, each blocking domain can be one base pair to tens of base pairs in length. Each blocking domain can be independently one, two, three, four, five, six, seven, eight, nine or ten nucleotides or base pairs in length.

In some embodiments, at least one of the blocking domain comprises a poly-monomer stretch. For example, the blocking domain comprises a stretch of polyA, polyT, polyC or polyG.

As used herein, a "blocking region" refers to a part of a template strand that along with a second template strand forms the blocking domain. Without limitations, the blocking region can independently comprise any desired nucleotide sequence or number of nucleotides. In other words, each blocking region can be independently of any length. For example, each blocking region can be one nucleotide to tens of nucleotides in length. Each blocking region can be independently one, two, three, four, five, six, seven, eight, nine or ten nucleotides in length. It is noted that a blocking region can be a single nucleotide.

At least one blocking region within a blocking domain may comprise a modified nucleotide that comprises a modification for cross-linking with another blocking region in the blocking domain.

As used herein, the term "cross-linking segment," refers to a part of the nucleic acid template that comprises a modified nucleotide that crosslinks to another oligonucleotide upon exposure to phase-changing stimulus (*e.g*. ultraviolet light, temperature change, pH change, etc.) and permits the assembly of the full nucleic acid template sequence. **FIG. 4A** provides an example of the crosslinking segments of the nucleic acid template.

Monomers and modified nucleotides for cross-linking of oligonucleotides are well known in the art. For example, photoreactive nucleotides are well known in the art for crosslinking oligonucleotides. Accordingly, a blocking region can comprise a photoreactive nucleotide, i.e., a nucleotide with a photoreactive group. Exemplary photoreactive nucleotides include, but are not limited to, 3-Cyanovinylcarbazole (CNVK) nucleotide; 5-bromo deoxycytosine; 5-iodo deoxycytosine; 5-bromo deoxyurdine; 5-iodo deoxyuridine; and nucleotides comprising an aryl azide (AB-dUMP), benzophenone (BP-dUMP), perfluorinated aryl azide (FAB-dUMP) or diazirine (DB-dUMP). A blocking region may comprise CNVK as the photoreactive nucleotide.

In some embodiments, the cross-linking segment comprises 3-cyanovinylcarbozole.

In some embodiments, the first and second blocking regions are covalently linked to each other; and/or the first or the second blocking region comprises a cross-linking segment with the other blocking region to form the first blocking domain, optionally wherein the crosslinking segment comprises 3-cyanovinylcabozole.

Exemplary secondary structure of the nucleic acid template and guide strands that make up the nucleic acid template are shown in **FIGs. 1A-2**. The secondary structure allows for the DNA polymerase to displace from the first guide strand onto the second guide strand, third guide strand, fourth guide strand, fifth guide strand, etc. until the output nucleic acid sequence is achieved. Different structure prediction models can produce different predicted structures, and even the same model can produce different predicted structures if different baseline parameter are used, *e.g*., temperature, ionic strength, etc.

That said, when a change of one nucleotide in a base-paired structure of a guide strand within the nucleic acid template is accompanied by a compensatory change in the complementary nucleotide that maintains the ability to base pair, the structure, and thereby the function of the guide strand can be maintained. That is, some guide strands can tolerate some degree of sequence change and still retain DNA polymerase displacement activity. Furthermore, a truncated or partial sequence of a nucleic acid template or guide strand as described herein can also retain displacement activity provided that the truncation does not alter intramolecular base-pairing necessary for the secondary structure of the guide strand or nucleic acid template. The Examples herein provide working demonstration of the nucleic acid templates that specifically displace the DNA polymerase.

It is contemplated that the reverse, complement, reverse complement, or truncated sequences of the nucleic acid templates described herein can also maintain secondary structure of the template. Secondary structure models can be used to predict the stability of the guide strands in the nucleic acid template. The maintenance or improvement in modified guide strands to produce a nucleic acid output sequence, *e.g.* a compensatory or non-compensatory change can be made on the basis of predicted structure should be tested experimentally.

In some embodiments, of the nucleic acid template one or more regions utilize a 3-letter code. As used herein, a "3-letter code" means the region comprises only three of the four nucleobases, i.e., only three of adenine, thymine/uracil, guanine, and cytosine, or modified versions thereof. For example, a 3-letter code can comprise or consists of nucleobases selected from one of the following:
(i) adenine, thymine/uracil, and guanine;
(ii) adenine, thymine/uracil, and cytosine;
(iii) adenine, guanine, and cytosine; or
(iv) thymine/uracil, guanine, and cytosine.

In some embodiments, the nucleic acid template or at least one of the first, second, third or fourth blocking domain comprises a nucleic acid modification; optionally wherein the nucleic acid modification is a modified nucleobase or a fluorophore on the 3' and/or 5'-end. The nucleic acid template and/or the synthesized nucleic acid sequence may comprise a nucleic acid modification. For example, at least one of a synthesis region, a junction domain, and/or a blocking domain may comprise a nucleic acid modification Exemplary nucleic acid modifications include, but are not limited to, nucleobase modifications, sugar modifications, inter-sugar linkage modifications, conjugates (*e.g*., ligands), and any combinations thereof.

Exemplary modified nucleobases include, but are not limited to, inosine, xanthine, hypoxanthine, nubularine, isoguanisine, tubercidine, and substituted or modified analogs of adenine, guanine, cytosine and uracil, such as 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine,7-deazaadenine, N6, N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N⁴-acetyl cytosine, 2-thiocytosine, N6-methyladenine, N6-isopentyladenine, 2-methylthio-N6-isopentenyladenine, N-methylguanines, or O-alkylated bases. Further purines and pyrimidines include those disclosed in U.S. Pat. No. 3,687,808, those disclosed in the Concise Encyclopedia of Polymer Science and Engineering, pages 858-859, Kroschwitz, J. I., ed. John Wiley & Sons, 1990, and those disclosed by Englisch et al., Angewandte Chemie, International Edition, 1991, 30, 613.

Modified nucleobase can be selected from the group consisting of inosine, xanthine, hypoxanthine, nubularine, isoguanisine, tubercidine, 2-(halo)adenine, 2-(alkyl)adenine, 2-(propyl)adenine, 2-(amino)adenine, 2-(aminoalkyll)adenine, 2-(aminopropyl)adenine, 2-(methylthio)-N⁶-(isopentenyl)adenine, 6-(alkyl)adenine, 6-(methyl)adenine, 7-(deaza)adenine, 8-(alkenyl)adenine, 8-(alkyl)adenine, 8-(alkynyl)adenine, 8-(amino)adenine, 8-(halo)adenine, 8-(hydroxyl)adenine, 8-(thioalkyl)adenine, 8-(thiol)adenine, N⁶-(isopentyl)adenine, N⁶-(methyl)adenine, N⁶, N⁶-(dimethyl)adenine, 2-(alkyl)guanine,2-(propyl)guanine, 6-(alkyl)guanine, 6-(methyl)guanine, 7-(alkyl)guanine, 7-(methyl)guanine, 7-(deaza)guanine, 8-(alkyl)guanine, 8-(alkenyl)guanine, 8-(alkynyl)guanine, 8-(amino)guanine, 8-(halo)guanine, 8-(hydroxyl)guanine, 8-(thioalkyl)guanine, 8-(thiol)guanine, N-(methyl)guanine, 2-(thio)cytosine, 3-(deaza)-5-(aza)cytosine, 3-(alkyl)cytosine, 3-(methyl)cytosine, 5-(alkyl)cytosine, 5-(alkynyl)cytosine, 5-(halo)cytosine, 5-(methyl)cytosine, 5-(propynyl)cytosine, 5-(propynyl)cytosine, 5-(trifluoromethyl)cytosine, 6-(azo)cytosine, N⁴-(acetyl)cytosine, 3-(3-amino-3-carboxypropyl)uracil, 5-ethynyl-2'-deoxyuridine, 2-(thio)uracil, 5-(methyl)-2-(thio)uracil, 5-(methylaminomethyl)-2-(thio)uracil, 4-(thio)uracil, 5-(methyl)-4-(thio)uracil, 5-(methylaminomethyl)-4-(thio)uracil, 5-(methyl)-2,4-(dithio)uracil, 5-(methylaminomethyl)-2,4-(dithio)uracil, 5-(2-aminopropyl)uracil, 5-(alkyl)uracil, 5-(alkynyl)uracil, 5-(allylamino)uracil, 5-(aminoallyl)uracil, 5-(aminoalkyl)uracil, 5-(guanidiniumalkyl)uracil, 5-(1,3-diazole-1-alkyl)uracil, 5-(cyanoalkyl)uracil, 5-(dialkylaminoalkyl)uracil, 5-(dimethylaminoalkyl)uracil, 5-(halo)uracil, 5-(methoxy)uracil, uracil-5-oxyacetic acid, 5-(methoxycarbonylmethyl)-2-(thio)uracil, 5-(methoxycarbonyl-methyl)uracil, 5-(propynyl)uracil, 5-(propynyl)uracil, 5-(trifluoromethyl)uracil, 6-(azo)uracil, dihydrouracil, N³-(methyl)uracil, 5-uracil (*i.e.,* pseudouracil), 2-(thio)pseudouracil,4-(thio)pseudouracil,2,4-(dithio)psuedouracil,5-(alkyl)pseudouracil, 5-(methyl)pseudouracil, 5-(alkyl)-2-(thio)pseudouracil, 5-(methyl)-2-(thio)pseudouracil, 5-(alkyl)-4-(thio)pseudouracil, 5-(methyl)-4-(thio)pseudouracil, 5-(alkyl)-2,4-(dithio)pseudouracil, 5-(methyl)-2,4-(dithio)pseudouracil, 1-substituted pseudouracil, 1-substituted 2(thio)-pseudouracil, 1-substituted 4-(thio)pseudouracil, 1-substituted 2,4-(dithio)pseudouracil, 1-(aminocarbonylethylenyl)-pseudouracil, 1-(aminocarbonylethylenyl)-2(thio)-pseudouracil, 1-(aminocarbonylethylenyl)-4-(thio)pseudouracil, 1-(aminocarbonylethylenyl)-2,4-(dithio)pseudouracil, 1-(aminoalkylaminocarbonylethylenyl)-pseudouracil, 1-(aminoalkylamino-carbonylethylenyl)-2(thio)-pseudouracil, 1-(aminoalkylaminocarbonylethylenyl)-4-(thio)pseudouracil, 1-(aminoalkylaminocarbonylethylenyl)-2,4-(dithio)pseudouracil, 1,3-(diaza)-2-(oxo)-phenoxazin-1-yl, 1-(aza)-2-(thio)-3-(aza)-phenoxazin-1-yl, 1,3-(diaza)-2-(oxo)-phenthiazin-1-yl, 1-(aza)-2-(thio)-3-(aza)-phenthiazin-1-yl, 7-substituted 1,3-(diaza)-2-(oxo)-phenoxazin-1-yl, 7-substituted 1-(aza)-2-(thio)-3-(aza)-phenoxazin-1-yl, 7-substituted 1,3-(diaza)-2-(oxo)-phenthiazin-1-yl, 7-substituted 1-(aza)-2-(thio)-3-(aza)-phenthiazin-1-yl, 7-(aminoalkylhydroxy)-1,3-(diaza)-2-(oxo)-phenoxazin-1-yl, 7-(aminoalkylhydroxy)-1-(aza)-2-(thio)-3-(aza)-phenoxazin-1-yl, 7-(aminoalkylhydroxy)-1,3-(diaza)-2-(oxo)-phenthiazin-1-yl, 7-(aminoalkylhydroxy)-1-(aza)-2-(thio)-3-(aza)-phenthiazin-1-yl, 7-(guanidiniumalkylhydroxy)-1,3-(diaza)-2-(oxo)-phenoxazin-1-yl, 7-(guanidiniumalkylhydroxy)-1-(aza)-2-(thio)-3-(aza)-phenoxazin-1-yl, 7-(guanidiniumalkyl-hydroxy)-1,3-(diaza)-2-(oxo)-phenthiazin-1-yl, 7-(guanidiniumalkylhydroxy)-1-(aza)-2-(thio)-3-(aza)-phenthiazin-1-yl, 1,3,5-(triaza)-2,6-(dioxa)-naphthalene, inosine, xanthine, hypoxanthine, nubularine, tubercidine, isoguanisine, inosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, 3-(methyl)isocarbostyrilyl, 5-(methyl)isocarbostyrilyl, 3-(methyl)-7-(propynyl)isocarbostyrilyl, 7-(aza)indolyl, 6-(methyl)-7-(aza)indolyl, imidizopyridinyl, 9-(methyl)-imidizopyridinyl, pyrrolopyrizinyl, isocarbostyrilyl, 7-(propynyl)isocarbostyrilyl, propynyl-7-(aza)indolyl, 2,4,5-(trimethyl)phenyl, 4-(methyl)indolyl, 4,6-(dimethyl)indolyl, phenyl, napthalenyl, anthracenyl, phenanthracenyl, pyrenyl, stilbenyl, tetracenyl, pentacenyl, difluorotolyl, 4-(fluoro)-6-(methyl)benzimidazole, 4-(methyl)benzimidazole, 6-(azo)thymine, 2-pyridinone, 5-nitroindole, 3-nitropyrrole, 6-(aza)pyrimidine, 2-(amino)purine, 2,6-(diamino)purine, 5-substituted pyrimidines, N²-substituted purines, N⁶-substituted purines, O⁶-substituted purines, substituted 1,2,4-triazoles, and any O-alkylated or N-alkylated derivatives thereof.

Exemplary sugar modifications include, but are not limited to, 2'-Fluoro, 3'-Fluoro, 2'-OMe, 3'-OMe, 2'-deoxy modifications, and acyclic nucleotides, e.g., peptide nucleic acids (PNA), unlocked nucleic acids (UNA) or glycol nucleic acid (GNA).

A nucleic acid modification can include replacement or modification of an inter-sugar linkage. Exemplary inter-sugar linkage modifications include, but are not limited to, phosphotriesters, methylphosphonates, phosphoramidate, phosphorothioates, methylenemethylimino, thiodiester, thionocarbamate, siloxane, N,N'-dimethylhydrazine (-CH2-N(CH3)-N(CH3)-), amide-3 (3'-CH₂-C(=O)-N(H)-5') and amide-4 (3'-CH₂-N(H)-C(=O)-5'), hydroxylamino, siloxane (dialkylsiloxxane), carboxamide, carbonate, carboxymethyl, carbamate, carboxylate ester, thioether, ethylene oxide linker, sulfide, sulfonate, sulfonamide, sulfonate ester, thioformacetal (3'-S-CH₂-O-5'), formacetal (3 '-O-CH₂-O-5'), oxime, methyleneimino, methykenecarbonylamino, methylenemethylimino (MMI, 3'-CH₂-N(CH₃)-O-5'), methylenehydrazo, methylenedimethylhydrazo, methyleneoxymethylimino, ethers (C3'-O-C5'), thioethers (C3'-S-C5'), thioacetamido (C3'-N(H)-C(=O)-CH₂-S-C5', C3'-O-P(O)-O-SS-C5', C3'-CH₂-NH-NH-C5', 3'-NHP(O)(OCH₃)-O-5' and 3'-NHP(O)(OCH₃)-O-5'.

Nucleic acid modifications can include peptide nucleic acids (PNA), bridged nucleic acids (BNA), morpholinos, locked nucleic acids (LNA), glycol nucleic acids (GNA), threose nucleic acids (TNA), or any other xeno nucleic acids (XNA) described in the art.

The nucleic acid template, a guide strand, and/or the synthesized nucleic acid sequence can be modified on the 3'- and/or 5'-end. For example, a label, fluorophore, tag, or a cap can be added to the 3' and/or 5'-end of the nucleic acid template, the guide strand, and/or the synthesized nucleic acid sequence.

The nucleic acid template, a guide strand, and/or the synthesized nucleic acid sequence can be modified with a linker or spacer, *e.g.*, at an internal position, on the 3'- and/or 5'-end. Without wishing to be bound by a theory, the linker or spacer can be used for linking the nucleic acid template, the guide strand, and/or the synthesized nucleic acid sequence with a moiety, such as a solid support or label. The linker or spacer can be selected from the group consisting of photocleavable linkers, hydrolyzable linkers, redox cleavable linkers, phosphate -based cleavable linkers, acid cleavable linkers, ester-based cleavable linkers, peptide-based cleavable linkers, and any combinations thereof. The cleavable linker can comprise a disulfide bond, a tetrazine-trans-cyclooctene group, a sulfhydryl group, a nitrobenzyl group, a nitoindoline group, a bromo hydroxycoumarin group, a bromo hydroxyquinoline group, a hydroxyphenacyl group, a dimethozybenzoin group, or any combinations thereof.

Any art-recognized photocleavable linker can be used. The cleavable linker can comprise a photocleavable linker. Generally, photocleavable linkers contain a photolabile functional group that is cleavable upon exposure to a light source (*e.g*., UV light) or specific wavelength. Non-limiting examples of photocleavable spacers can be found, for example, in US Patent Nos. 6,589,736 B1; 7,622,279 B2; 9,371,348 B2; 7,547,530 B2; and 7,057,031 B2; and PCT Publication No. WO2014200767.

The nucleic acid template, a guide strand, and/or the synthesized nucleic acid sequence can be modified with a detectable label, *e.g*., at an internal position, on the 3'- and/or 5'-end. Without wishing to be bound by a theory, such a detectable label can facilitate detection. As used herein, the term "detectable label" refers to a composition capable of producing a detectable signal indicative of the presence of a target. Detectable labels include any composition detectable by spectroscopic, photochemical, biochemical, immunochemical, electrical, optical or chemical means. Suitable labels include fluorescent molecules, radioisotopes, nucleotide chromophores, enzymes, substrates, chemiluminescent moieties, bioluminescent moieties, and the like. As such, a label is any composition detectable by spectroscopic, photochemical, biochemical, immunochemical, electrical, optical or chemical means.

A wide variety of fluorescent reporter dyes are known in the art. Typically, the fluorophore is an aromatic or heteroaromatic compound and can be a pyrene, anthracene, naphthalene, acridine, stilbene, indole, benzindole, oxazole, thiazole, benzothiazole, cyanine, carbocyanine, salicylate, anthranilate, coumarin, fluorescein, rhodamine or other like compound.

Exemplary fluorophores include, but are not limited to, 1,5 IAEDANS; 1,8-ANS ; 4-Methylumbelliferone; 5-carboxy-2,7-dichlorofluorescein; 5-Carboxyfluorescein (5-FAM); 5-Carboxynapthofluorescein (pH 10); 5-Carboxytetramethylrhodamine (5-TAMRA); 5-FAM (5-Carboxyfluorescein); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITSA; Aequorin (Photoprotein); Alexa Fluor 350^{™}; Alexa Fluor 430^{™}; Alexa Fluor 488^{™}; Alexa Fluor 532^{™}; Alexa Fluor 546^{™}; Alexa Fluor 568^{™}; Alexa Fluor 594^{™}; Alexa Fluor 633^{™}; Alexa Fluor 647^{™}; Alexa Fluor 660^{™}; Alexa Fluor 680^{™}; Alizarin Complexon; Alizarin Red; Allophycocyanin (APC); AMC, AMCA-S; AMCA (Aminomethylcoumarin); AMCA-X; Aminoactinomycin D; Aminocoumarin; Anilin Blue; Anthrocyl stearate; APC-Cy7; APTS; Astrazon Brilliant Red 4G; Astrazon Orange R; Astrazon Red 6B; Astrazon Yellow 7 GLL; Atabrine; ATTO-TAG^{™} CBQCA; ATTO-TAG^{™} FQ; Auramine; Aurophosphine G; Aurophosphine; BAO 9 (Bisaminophenyloxadiazole); BCECF (high pH); BCECF (low pH); Berberine Sulphate; Beta Lactamase; BFP blue shifted GFP (Y66H); BG-647; Bimane; Bisbenzamide; Blancophor FFG; Blancophor SV; BOBO^{™} -1; BOBO^{™} -3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy Fl; Bodipy FL ATP; Bodipy Fl-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO^{™} -1; BO-PRO^{™} -3; Brilliant Sulphoflavin FF; Calcein; Calcein Blue; Calcium Crimson^{™}; Calcium Green; Calcium Green-1 Ca²⁺ Dye; Calcium Green-2 Ca²⁺; Calcium Green-SN Ca²⁺; Calcium Green-C18 Ca²⁺; Calcium Orange; Calcofluor White; Carboxy-X-rhodamine (5-ROX); Cascade Blue^{™}; Cascade Yellow; Catecholamine; CFDA; CFP - Cyan Fluorescent Protein; Chlorophyll; Chromomycin A; CMFDA; Coelenterazine ; Coelenterazine cp; Coelenterazine f; Coelenterazine fcp; Coelenterazine h; Coelenterazine hcp; Coelenterazine ip; Coelenterazine O; Coumarin Phalloidin; CPM Methylcoumarin; CTC; Cy2^{™}; Cy3.1 8; Cy3.5^{™}; Cy3^{™}; Cy5.1 8; Cy5.5^{™}; Cy5^{™}; Cy7^{™}; Cyan GFP; cyclic AMP Fluorosensor (FiCRhR); d2; Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3; DCFDA; DCFH (Dichlorodihydrofluorescein Diacetate); DDAO; DHR (Dihydorhodamine 123); Di-4-ANEPPS; Di-8-ANEPPS (non-ratio); DiA (4-Di-16-ASP); DIDS; Dihydorhodamine 123 (DHR); DiO (DiOC18(3)); DiR; DiR (DiIC18(7)); Dopamine; DsRed; DTAF; DY-630-NHS; DY-635-NHS; EBFP; ECFP; EGFP; ELF 97; Eosin; Erythrosin; Erythrosin ITC; Ethidium homodimer-1 (EthD-1); Euchrysin; Europium (III) chloride; Europium; EYFP; Fast Blue; FDA; Feulgen (Pararosaniline); FITC; FL-645; Flazo Orange; Fluo-3; Fluo-4; Fluorescein Diacetate; Fluoro-Emerald; Fluoro-Gold (Hydroxystilbamidine); Fluor-Ruby; FluorX; FM 1-43^{™}; FM 4-46; Fura Red^{™} (high pH); Fura-2, high calcium; Fura-2, low calcium; Genacryl Brilliant Red B; Genacryl Brilliant Yellow 10GF; Genacryl Pink 3G; Genacryl Yellow 5GF; GFP (S65T); GFP red shifted (rsGFP); GFP wild type, non-UV excitation (wtGFP); GFP wild type, UV excitation (wtGFP); GFPuv; Gloxalic Acid; Granular Blue; Haematoporphyrin; Hoechst 33258; Hoechst 33342; Hoechst 34580; HPTS; Hydroxycoumarin; Hydroxystilbamidine (FluoroGold); Hydroxytryptamine; Indodicarbocyanine (DiD); Indotricarbocyanine (DiR); Intrawhite Cf; JC-1; JO-JO-1; JO-PRO-1; LaserPro; Laurodan; LDS 751; Leucophor PAF; Leucophor SF; Leucophor WS; Lissamine Rhodamine; Lissamine Rhodamine B; LOLO-1; LO-PRO-1; Lucifer Yellow; Mag Green; Magdala Red (Phloxin B); Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant Iavin E8G; Oregon Green^{™}; Oregon Green 488-X; Oregon Green^{™} 488; Oregon Green^{™} 500; Oregon Green^{™} 514; Pacific Blue; Pararosaniline (Feulgen); PE-CyS; PE-Cy7; PerCP; PerCP-Cy5.5; PE-TexasRed (Red 613); Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 ; PKH67; PMIA; Pontochrome Blue Black; POPO-1; POPO-3; PO-PRO-1; PO-PRO-3; Primuline; Procion Yellow; Propidium Iodid (PI); PyMPO; Pyrene; Pyronine; Pyronine B; Pyrozal Brilliant Flavin 7GF; QSY 7; Quinacrine Mustard; Resorufin; RH 414; Rhod-2; Rhodamine; Rhodamine 110; Rhodamine 123; Rhodamine 5 GLD; Rhodamine 6G; Rhodamine B 540; Rhodamine B 200 ; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycoerythrin (PE); red shifted GFP (rsGFP, S65T); S65A; S65C; S65L; S65T; Sapphire GFP; Serotonin; Sevron Brilliant Red 2B; Sevron Brilliant Red 4G; Sevron Brilliant Red B; Sevron Orange; Sevron Yellow L; sgBFP^{™}; sgBFP^{™} (super glow BFP); sgGFP^{™}; sgGFP^{™} (super glow GFP); SITS; SITS (Primuline); SITS (Stilbene Isothiosulphonic Acid); SPQ (6-methoxy-N-(3-sulfopropyl)-quinolinium); Stilbene; Sulphorhodamine B can C; Sulphorhodamine G Extra; Tetracycline; Tetramethylrhodamine ; Texas Red^{™}; Texas Red-X^{™} conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; TriColor (PE-Cy5); TRITC (TetramethylRodamineIsoThioCyanate); True Blue; TruRed; Ultralite; Uranine B; Uvitex SFC; wt GFP; WW 781; XL665; X-Rhodamine; XRITC; Xylene Orange; Y66F; Y66H; Y66W; Yellow GFP; YFP; YO-PRO-1; YO-PRO-3; YOYO-1; and YOYO-3. Many suitable forms of these fluorescent compounds are available and can be used.

Other exemplary detectable labels include luminescent and bioluminescent markers (*e.g*., biotin, luciferase (*e.g*., bacterial, firefly, click beetle and the like), luciferin, and aequorin), radiolabels (*e.g*., 3H, 125I, 35S, 14C, or 32P), enzymes (*e.g*., galactosidases, glucorinidases, phosphatases (*e.g*., alkaline phosphatase), peroxidases (*e.g.*, horseradish peroxidase), and cholinesterases), and calorimetric labels such as colloidal gold or colored glass or plastic (*e.g*., polystyrene, polypropylene, and latex) beads. Patents teaching the use of such labels include U.S. Pat. Nos. 3,817,837, 3,850,752, 3,939,350, 3,996,345, 4,277,437, 4,275,149, and 4,366,241.

Means of detecting such labels are well known to those of skill in the art. Thus, for example, radiolabels can be detected using photographic film or scintillation counters, fluorescent markers can be detected using a photo-detector to detect emitted light. Enzymatic labels are typically detected by providing the enzyme with an enzyme substrate and detecting the reaction product produced by the action of the enzyme on the enzyme substrate, and calorimetric labels can be detected by visualizing the colored label.

The detectable label may be a fluorophore or a quantum dot. Without wishing to be bound by a theory, using a fluorescent reagent can reduce signal-to-noise in the imaging/readout, thus maintaining sensitivity.

A label can be configured to include a "smart label", which is undetectable when conjugated with the nucleic acid template, the guide strand, and/or the synthesized nucleic acid sequence, but produces a color change when released.

Acrydite modifications can also be made to the nucleic acid template, a guide strand, and/or the synthesized nucleic acid sequence. Acrydite modifications can permit the oligonucleotides to be used in reactions with nucleophiles such as thiols (e.g, microarrays) or incorporated into gels (e.g, polyacrylamide). Accordingly, the nucleic acid template, a guide strand, and/or the synthesized nucleic acid sequence may comprise one or more acrydite nucleosides. The acrydite nucleoside can be at the 3'- end, 5-end, and/or at an internal position of the nucleic acid template, the guide strand, and/or the synthesized nucleic acid sequence.

Any modifications to the nucleic acid template, a guide strand, and/or the synthesized nucleic acid sequences of the invention that permit purification, extraction, quantification of expression, binding, electrophoresis, and the like, can be made.

The nucleic acid template can comprise any desired number of crossjunctions, i.e., the nucleic acid template can comprise more than two guide strands. For example, the nucleic acid template comprises two cross-junctions, e.g., the nucleic acid template comprises three guide strands. Thus, the second guide strand further may comprise at its 5'end in 3' to 5' direction a third junction domain and a third blocking region, and the nucleic acid template further comprises a third guide strand. The third guide strand may comprise in 3' to 5' direction a fourth blocking region, a fourth junction domain and a fifth synthesis region. The third junction domain may comprise a nucleotide sequence substantially identical to a nucleotide sequence of the fifth synthesis region, wherein the third junction domain and fourth junction domain are substantially complementary to each other and form a double-stranded region, and wherein the third blocking region and the fourth blocking region together form a second blocking domain that blocks strand displacement activity of a polymerase.

In some embodiments, the third guide strand further comprises a sixth synthesis region at the 5' end of the fifth synthesis region; optionally wherein the third guide strand further comprises at its 5'end in 3' to 5' direction a fifth junction domain and a fifth blocking region, and the nucleic acid template further comprises a fourth guide strand comprising in 3' to 5' direction a sixth blocking region, a sixth junction domain and a seventh synthesis region, wherein the fifth junction domain comprises a nucleotide sequence substantially identical to a nucleotide sequence of the seventh synthesis region, and the fifth junction domain and sixth junction domain are substantially complementary to each other and form a double-stranded region, and wherein the fifth blocking region and the sixth blocking region together form a third blocking domain that blocks strand displacement activity of a polymerase.

In some embodiments, the third and the fourth blocking regions are covalently linked to each other; and/or the third or the fourth blocking region comprises a cross-linking segment with the other blocking region to form the second blocking domain, optionally wherein the cross-linking segment comprises 3-cyanovinylcabozole.

The nucleic acid template may comprise three cross-junctions, e.g., the nucleic acid template comprises four guide strands.

In some embodiments, the fourth guide strand further comprises an eighth synthesis region at the 5' end of the seventh synthesis region; optionally wherein the fourth guide strand further comprises at its 5'end in 3' to 5' direction a seventh junction domain and a seventh blocking region, and the nucleic acid template further comprises a fifth guide strand comprising in 3' to 5' direction an eighth blocking region, an eighth junction domain and a ninth synthesis region, wherein the seventh junction domain comprises a nucleotide sequence substantially identical to a nucleotide sequence of the ninth synthesis region, and the seventh junction domain and eighth junction domain are substantially complementary to each other and form a double-stranded region, and wherein the seventh blocking region and the eighth blocking region together form a fourth blocking domain that blocks strand displacement activity of a polymerase.

In some embodiments, the fifth and sixth blocking regions are covalently linked to each other and/or the fifth or the sixth blocking region comprises a cross-linking segment with the other blocking region to form the third blocking domain, optionally wherein the crosslinking segment comprises 3-cyanovinylcabozole.

The nucleic acid template may comprise four cross-junctions, *e.g*., the nucleic acid template comprises five guide strands.

In some embodiments, the seventh and eighth blocking regions are covalently linked to each other; and/or the seventh or the eighth blocking region comprises a cross-linking segment with the other blocking region to form the fourth blocking domain, optionally wherein the cross-linking segment comprises 3-cyanovinylcabozole.

All guide strands in the nucleic acid template can comprise a substantially identical nucleotide sequence in the synthesis region in the 5'-side of the guide strands. All guide strands in the nucleic acid template can comprise a substantially identical nucleotide sequence in the synthesis region in the 3'-side of the guide strands.

In some embodiments, the nucleic acid template, is conjugated to a solid support. The nucleic acid template, guide strand, or portion thereof may be immobilized, conjugated to, or linked to a solid support or a substrate. The nucleic acid template, guide strand, or portion thereof may be immobilized on a substrate surface. The nucleic acid template, guide strand, or portion thereof represents information, date, or spatial information. It is noted that the nucleic acid template, guide strand, or portion thereof can be immobilized, conjugate to, or linked to the solid support or substrate covalently or non-covalently.

The nucleic acid template, guide strand, or portion thereof may be immobilized in a predetermined pattern. The predetermined pattern may be a geometric shape, a square, a circle, or triangle. The predetermined pattern may comprise repeating elements. The predetermined pattern may be asymmetrical or symmetrical. The predetermined pattern may comprise spatial information and/or special information.

Without limitations, the solid support or substrate can exist in the form of a platform, column, filter or sheet, dish, a microfluidic capture device, capillary tube, electrochemical responsive platform, scaffold, cartridge, resin, matrix, bead, or another solid support known in the art. The substrate can also comprise a biological material. Biological materials are known in the art. Non-limiting examples of biological materials include tissues, tissue sections, engineered tissues, cells, patient derived cells, primary cells, organoids, extracellular matrix, 3D biological organs, dissociated cells, live cells, fixed cells, vesicles, droplets, liposomes, etc.

The solid support or substrate may comprise materials that include, but are not limited to, a polymer, metal, ceramic, gels, paper, or glass. The materials of the solid support can further comprise, as non-limiting examples, polystyrene, agarose, gelatin, alginate, iron oxide, stainless steel, gold nanobeads or particles, copper, silver chloride, polycarbonate, polydimethylsiloxane, polyethylene, acrylonitrile butadiene styrene, cyclo-olefin polymers or cyclo-olefin copolymers, or Sepharose^{™} resin.

The substrate may be a hydrogel. The hydrogel may be a compressed hydrogel. A hydrogel can be naturally occurring, derived from a natural source, or derived from a synthetic source. A hydrogel can be any water-swollen and cross-linked polymeric material produced by a reaction of one or more monomers. A hydrogel can be a polymeric material that is capable of expanding to retain a significant fraction of water within its structure without dissolving into the aqueous solution. A hydrogel can also be any shrinkable material, *e.g*., heat-shrinkable plastics, viscoelastic foam, memory foam.

The solid support or substrate can further comprise a magnetoresponsive element such as a magnetoresponsive bead. The magnetoresponsive element or bead may be in the form of a sphere, cube, rectangle, cylinder, cone, or any other shape described in the art.

The magnetoresponsive element may comprise magnetite, iron (III) oxide, samarium-cobalt, terfenol-D, or any other magnetic element described in the art.

### Synthesis of nucleic acid template

In a second aspect, the present invention is a method for forming a nucleic acid template , the method comprising annealing or hybridizing a first guide strand and a second guide strand, wherein: (a) the first guide strand comprises in 3' to 5' direction a first synthesis region, a second synthesis region, a first junction domain, and a first blocking region; and (b) the second guide strand comprises in 3' to 5' direction a second blocking region, a second junction domain and a third synthesis region, wherein the first junction domain comprises a nucleotide sequence substantially identical to a nucleotide sequence of the third synthesis region, and the first junction domain and second junction domain are substantially complementary to each other and form a double-stranded region, and wherein the first blocking region and the second blocking region together form a blocking domain that blocks strand displacement activity of a polymerase.

In some embodiments, the first or the second blocking region comprises a crosslinking segment with the other blocking region to form the first blocking domain. The method may further comprise forming the cross-link.

The nucleic acid templates and methods described herein can be used for synthesis of arbitrary length prescribed sequences. An example is shown in **FIG. 2****.** As shown in **FIG. 2****,** by hybridizing multiple guide strands together, cross-junction synthesis reactions can be cascaded to form longer sequences. Each junction between guide strands shows the same domain motifs, whereby the strand domains copied on the 3' end of the growing strand before reaching a stopper (**b, c, d, e**) can reach across the junction and bind to the exposed complementary sequence (**b*, c*, d*, e***) on the next guide strand. Arbitrary sequences can be added in the template regions between the motif domains (shown in gray) to enable longer sequences to be assembled.

The nucleic acid templates and methods described herein can be used for synthesis of polymeric sequences of prescribed length. For example, the compositions and methods described herein can be used for synthesizing specific numbers of repeated sequences through the use of programmable unique hybridization domains that fix the length of the template concatemer, which has been a long-standing challenge. An example is shown in **FIG. 3****.** As shown in **FIG. 3****,** by harnessing the specificity of DNA self-assembly, unique binding sequences promote assemblies of prescribed length, even if the domains being synthesized are identical.

Cross-junctions can be assembled by utilizing a photoreactive inter-strand crosslinking base, which can serve the dual purpose of linking two cross-junction strands together and acting as a stopper for polymerase synthesis (**FIG. 4**). Cross-junctions can be assembled iteratively through cycles of hybridization, crosslinking and washing. Crossjunctions can also be assembled and spatially addressed on a surface, and subsequent synthesis steps can still be performed on the surface.

Assembling a library of orthogonal cross-junction arrays on a surface can enable multiplexed synthesis of orthogonal, and arbitrary length DNA of prescribed sequences.

In one example, 3-Cyanovinylcarbazole (CNVK) (Vieregg *et al.,* 2013) base modification and a UV light source to crosslink DNA junctions together is used. Strand design can utilize a set of two or more hybridization domains (**1** and **1*, 2** and **2*** in current implementation), an internal barcode sequence (b1, b2, etc....) that will serve as the growing sequence chain, and the CNVK base cross-linker (**FIG. 4A**, cyan circle).

### Nucleic acid synthesis

In a third aspect, the present invention is a method for synthesizing a nucleic acid sequence, the method comprising: providing or obtaining a nucleic acid template of any of claims 1 to 11; annealing or hybridizing a primer, if not already annealed, to the nucleic acid template; and extending a nucleotide sequence using a DNA polymerase having strand displacement activity from the 3'-terminus of the primer; optionally wherein the method further comprises amplifying the nucleic acid sequence or further comprises sequencing the synthesized nucleic acid sequence.

The method comprises annealing or hybridizing a primer to the nucleic acid template. For example, the primer is annealed or otherwise hybridized to a guide strand in the nucleic acid template. The primer can be annealed or hybridized to a guide strand of the nucleic acid template prior to assembling the full length nucleic acid.

The nucleic acid template for use in the method of synthesizing a nucleic acid described herein can be fully assembled prior to the synthesis. For example, as shown in **FIGs. 5-12** and 14-19A, all the desired guide strands can be annealed or hybridized together, to form the full length template, prior to extension from the primer. Thus, the method for synthesizing a nucleic acid may comprise annealing or hybridizing the guide strands to form the full length nucleic acid template prior to extending the nucleotide sequence from the 3'-end of the primer. For example, all the guide strands can be added in an initial reaction mixture and allowed to anneal or hybridize prior to the extension step.

Alternatively, the synthesis can be continuous. For example, the guide strands can be added consecutively to the synthesis reaction over a period of time under conditions that permit synthesis of the desired nucleic acid sequence across the junctions. Generally, the synthesis starts by extending a nucleotide sequence from the 3'-end of a primer annealed or hybridized to a guide strand. The reaction is substantially free of other guide strands of the full length template. After a period of time, *e.g.,* 15 seconds, 30 seconds, 45 seconds, 1 minutes, 2 minutes, 3 minutes, 4 minutes, 5 minutes or more, the next guide strand is added. This can be continued until the desired full length template has been assembled.

As shown in the example in **FIG. 21A****,** the synthesis starts with annealing or hybridizing a first guide strand with a primer. The first guide strand comprises in a 3' to 5' direction a first synthesis region, a second synthesis region, a junction domain and a blocking region. After it has annealed or hybridized to the first guide strand, the primer is extended from its 3'-end. After a period of time, e.g., e.g., 5 seconds, 10 seconds, 15 seconds, 30 seconds, 45 seconds, 1 minutes, 2 minutes, 3 minutes, 4 minutes, 5 minutes, 10 minutes, 15 minutes, 20 minutes or more, additional new guide strands can be added consecutively to the synthesis reaction.

It is noted that after a new guide strand is added to the synthesis reaction, the new guide strand is allowed to anneal or otherwise hybridize to a guide strand already in the synthesis reaction. Optionally, the complementary blocking regions are covalently crosslinked, e.g., by photo-crosslinking. The synthesis is allowed to continue for a period of time, e.g., 5 seconds, 10 seconds, 15 seconds, 30 seconds, 45 seconds, 1 minutes, 2 minutes, 3 minutes, 4 minutes, 5 minutes, 10 minutes, 15 minutes, 20 minutes or more, before the next guide strand is added to the reaction.

Generally, each additional guide strand comprises in a 3' to 5' direction, a first blocking region, a first junction domain, a first synthesis region, a second synthesis region, a second junction domain, and a second blocking region. The first new guide strand added to the synthesis reaction has the following characteristics: the first blocking region of the new guide strand and the blocking region of the first guide strand together form a blocking domain that blocks strand displacement activity of a polymerase; the first junction domain of the new guide strand comprises a nucleotide sequence substantially complementary to a nucleotide sequence of the junction domain of the first guide strand to form a double-stranded region; and the first synthesis region of the new guide strand comprises a nucleotide sequence substantially identical to a nucleotide sequence of the junction domain of the first guide strand.

Each additional new guide strand added after the first new guide strand has the following characteristics: the first blocking region of the new guide strand and the second blocking region of the last guide strand added to the synthesis reaction together form a blocking domain that blocks strand displacement activity of a polymerase; the first junction domain of the new guide strand comprises a nucleotide sequence substantially complementary to a nucleic acid sequence of the second junction domain of the last guide strand added to the synthesis reaction to form a double-stranded region; and the first synthesis region of the new guide strand comprises a nucleotide sequence substantially identical to a nucleic acid sequence of the second junction domain of the last guide strand added to the synthesis reaction.

The number of additional guide strands that can be added is not limited. For example, 1, 2, 3, 4, 5, 6, 7, 8, 9, 10, 11, 12, 13, 14, 15, 16, 17, 18, 19, 20, 21, 22, 23, 24, 25 or more additional guide strands can be added. 75, 100, 150, 200, 250 or more additional guide strands can be added.

The method may further comprise adding a terminal guide strand to the synthesis reaction. The terminal guide strand may comprise in a 3' to 5' direction a blocking region, a junction domain, a first synthesis region, a second synthesis region, a second junction domain, and a second blocking region, wherein the first blocking region of the terminal guide stand and the second blocking region of the last guide strand added to the synthesis reaction together form a blocking domain that blocks strand displacement activity of a polymerase; the first junction domain of the terminal guide strand comprises a nucleotide sequence substantially complementary to a nucleic acid sequence of the second junction domain of the last guide strand added to the synthesis reaction to form a double-stranded region; and the first synthesis region of the terminal guide strand comprises a nucleotide sequence substantially identical to a nucleic acid sequence of the second junction domain of the last guide strand added to the synthesis reaction. The terminal guide strand may optionally comprise a primer sequence.

If no additional guide strands are added to the synthesis, the terminal guide may have the following characteristics: the blocking region of the terminal guide strand and the blocking region of the first guide strand together form a blocking domain that blocks strand displacement activity of a polymerase; the junction domain of the terminal guide strand comprises a nucleotide sequence substantially complementary to a nucleic acid sequence of the junction domain of the first guide strand to form a double-stranded region; and the first synthesis region of the terminal guide strand comprises a nucleotide sequence substantially identical to a nucleic acid sequence of the junction domain of the first guide strand added to the synthesis reaction.

The terminal guide strand may comprise a primer sequence at its 5'-end.

As used herein, the term "primer" is used to describe a sequence of DNA (or RNA) that is paired with one strand of DNA and provides a free 3'-OH at which a DNA polymerase starts synthesis of a deoxyribonucleotide chain. Preferably, the primer is composed of an oligonucleotide. The exact lengths of the primers will depend on many factors, including temperature and source of primer. For example, depending on the complexity of the target sequence, the oligonucleotide primer typically contains 15-25 or more nucleotides, although it may contain fewer nucleotides. Short primer molecules generally require cooler temperatures to form sufficiently stable hybrid complexes with template.

A "polymerase" refers to an enzyme that performs template-directed synthesis of polynucleotides, e.g., DNA and/or RNA. The term encompasses both the full length polypeptide and a domain that has polymerase activity. DNA polymerases are well-known to those skilled in the art, including but not limited to DNA polymerases isolated or derived from *Pyrococcus furiosus, Thermococcus litoralis,* and *Thermotoga maritime,* or modified versions thereof. Additional examples of commercially available polymerase enzymes include, but are not limited to: Klenow fragment (New England Biolabs^{®} Inc.), Taq DNA polymerase (QIAGEN), 9° N^{™} DNA polymerase (New England Biolabs^{®} Inc.), Deep Vent^{™} DNA polymerase (New England Biolabs^{®} Inc.), Manta DNA polymerase (Enzymatics^{®}), Bst DNA polymerase (New England Biolabs^{®} Inc.), and phi29 DNA polymerase (New England Biolabs^{®} Inc.). Polymerases include both DNA-dependent polymerases and RNA-dependent polymerases such as reverse transcriptase. At least five families of DNA-dependent DNA polymerases are known, although most fall into families A, B and C. There is little or no sequence similarity among the various families. Most family A polymerases are single chain proteins that can contain multiple enzymatic functions including polymerase, 3' to 5' exonuclease activity and 5' to 3' exonuclease activity. Family B polymerases typically have a single catalytic domain with polymerase and 3' to 5' exonuclease activity, as well as accessory factors. Family C polymerases are typically multi-subunit proteins with polymerizing and 3' to 5' exonuclease activity. In *E. coli,* three types of DNA polymerases have been found, DNA polymerases I (family A), II (family B), and III (family C). In eukaryotic cells, three different family B polymerases, DNA polymerases α, δ, and ε, are implicated in nuclear replication, and a family A polymerase, polymerase γ, is used for mitochondrial DNA replication. Other types of DNA polymerases include phage polymerases. Similarly, RNA polymerases typically include eukaryotic RNA polymerases I, II, and III, and bacterial RNA polymerases as well as phage and viral polymerases. RNA polymerases can be DNA-dependent and RNA-dependent.

It is noted that reagents, such as strand displacing DNA or RNA polymerases, and methods for synthesizing nucleic acid sequences from nucleic acid templates are well known in the art and are amenable to the invention. See, for example, US20050277146A1, US20100035303A1, and WO2006030455A1.

The method may further comprise amplifying the nucleic acid sequence. As used herein, the term "amplifying" refers to a step of submitting a nucleic acid sequence to conditions sufficient to allow for amplification of a polynucleotide if all of the components of the reaction are intact. Components of an amplification reaction include, e.g., primers, a polynucleotide template, polymerase, nucleotides, and the like. The term "amplifying" typically refers to an "exponential" increase in target nucleic acid. However, "amplifying" as used herein can also refer to linear increases in the numbers of a select target sequence of nucleic acid, such as is obtained with cycle sequencing. Methods of amplifying and synthesizing nucleic acid sequences are known in the art. For example, see US Patent Nos. 7,906.282, 8,367,328, 5,518,900, 7,378,262, 5,476,774, and 6,638,722.

Amplifying the nucleic acid sequence may comprise a polymerase chain reaction (PCR). PCR is well known to those of skill in the art; see, e.g., U.S. Patent Nos. 4,683,195 and 4,683,202; and PCR Protocols: A Guide to Methods and Applications, Innis et al., eds, 1990. Exemplary PCR reaction conditions typically comprise either two or three step cycles. Two step cycles have a denaturation step followed by a hybridization/elongation step. Three step cycles comprise a denaturation step followed by a hybridization step followed by a separate elongation step.

The amplification step may include additional polynucleotide sequences or templates with hairpins that are orthogonal. Without wishing to be bound by a theory, such additional DNA hairpins can reduce or correct for off-target reactions. For example, when a three-letter code is used, these additional hairpins comprising sequences or templates can serve to soak up the trace amounts of unwanted nucleotide that can be present in some samples.

The guide strands may be added in an initial reaction mixture. The synthesis may be continuous. In continuous synthesis, the guide strands are each added consecutively under conditions that permit synthesis of the desired nucleic acid across the junctions. See for example, **FIG. 21A** in the working examples, which provides a schematic representation of a method described herein.

To complete a round of continuous nucleic acid synthesis as described herein, a terminal barcode strand can be used. As used herein, a "terminal barcode strand" comprises (i) one or more substantially identical binding domains to one template strand; and optionally, (ii) a different barcode; and optionally (iii) a PCR primer sequence on the 5' end to enable complete synthesis products to be exponentially amplified. Specifically, the optional barcode of the terminal barcode strand is not complementary to the template strand. Without limitations, the terminal barcode strand can be added to the 3' or 5' end of the template nucleic acid. The terminal barcode strand can further comprise a label or a fluorophore.

Provided herein is a reaction mixture comprising a nucleic acid template described.

In some embodiments, the nucleic acid template further comprises a primer annealed or hybridized to the one of the guide strands. In another embodiment, the nucleic acid template further comprises a primer annealed or hybridized to the first guide strand. The nucleic acid template may further comprise a primer annealed or hybridized to the second guide strand, third guide strand, fourth guide strand, fifth guide strand, sixth guide strand, seventh guide strand, etc.

The reaction mixture may further comprise nucleotide triphosphates or deoxynucleotide triphosphates. The reaction mixture may not include one of adenosine, thymidine/uridine, cytosine or guanosine triphosphate or deoxynucleotide triphosphate.

The reaction mixture may further comprise a DNA or RNA polymerase.

the reaction mixture may further comprise a buffer or salt for nucleic acid synthesis. It is contemplated that buffer used in the reaction mixture is chosen that permit the stability of the nucleic acid template and desired nucleic acid sequence. Methods of choosing such buffers are known in the art and can also be chosen for their properties in various conditions including pH or temperature of the reaction being performed.

the reaction mixture may comprise components that can be utilized to create the guide strands by addition of junction regions onto existing nucleic acid strands enzymatically or chemically. For example, a reaction mixture that can be utilized to create the guide strands by addition of blocking regions onto existing nucleic acid strands enzymatically or chemically.

Disclosed herein is a kit comprising the nucleic acid template as provided herein. Disclosed herein, such kits are intended for therapeutic application. Disclosed herein, such kits are intended for research use.

Disclosed herein, the kit further comprises nucleotide triphosphates or deoxynucleotide triphosphates.

Disclosed herein, the kit further comprises a DNA or RNA polymerase.

Disclosed herein, the kit further comprises a buffer or salt for nucleic acid synthesis.

Disclosed herein, the kit further comprises solid supports as provided herein to isolate specific sequences.

Disclosed herein is a kit comprising components or reaction mixture for creating the nucleic acid template described herein from nucleic acid sequences (partial guide strands) by addition of junction regions and/or blocking regions onto existing nucleic acid strands enzymatically or chemically.

Disclosed herein, the kit further comprises instructions for use.

Methods of purification and analysis of nucleic acids are known to those skilled in the art. Non-limiting examples of methods to characterize the nucleic acid synthesis include liquid chromatography, mass spectrometry, next generation sequencing, polymerase chain reaction (PCR), gel electrophoresis, or any other method of identifying nucleoside sequences, secondary structures, chemical composition, expression, thermodynamics, binding, or function.

### Exemplary Applications

The nucleic acid template and methods of the invention are fundamentally enabling for several different applications. Non-limiting examples include, but are not limited to, those described below.

*Proximity and molecular distance measurements.* Because the synthesis step happens isothermally, and the hybridization kinetics can be tuned to also happen under mild (non-denaturing) conditions, the proximity of biomolecules can be determined and create records indicative of distance information between labeled biomolecules. This has applications in distance/proximity measurements of DNA/RNA/proteins and other biomolecules of interest, structure determination of DNA, RNA, protein, and RNA-protein complexes. This could be further used as a method for fingerprinting of proteins and other biomolecules, if the synthesis reactions are programmed to produce relatively distinct record patterns from other targets.

*Long strand synthesis and DNA assembly.* Because the assembly and synthesis steps can be decoupled, assembly of larger sequences than is possible with other methods may be performed.

*Combinatorial synthesis for library generation.* The template concatemerization can be designed to produce a programmable combinatorial set of sequences (**FIG. 18**). Multiple options of intercalating domains may be desired in a sequence library, and this can be implemented by simply including the relevant guide strands with each of the options included at the desired relative frequency. For example, 10 options for each of 5 different domains would require 10 * 5 = 50 guide strands in place of the usual 5 for fixed domains, but would enable the creation of 10^5 = 100,000 possible sequences produced. In general, for positions *p1, p2,* ..., *pn,* each with a number of possibilities *x1, x3,* ..., *xn,* the total number of strands required for those positions would be *x1* + *x2* + ... + *xn,* and they can produce a library of size *x1* * *x3* * ... ** xn.*

*Parallel synthesis of multiple orthogonal sequences.* Multiple orthogonal cross-junction synthesis reaction can be performed together in the same reaction solution. These may or may not use the same primer or intercalating sequences.

*In-situ synthesis.* The synthesis step is performed in an aqueous, isothermal environment, allowing *in-situ* synthesis of arbitrary programmable strands within biological samples or biological materials.

*Constructing identity barcodes (unique molecular identifiers, batch numbers, indices).* The cross-junction synthesis can be used to create unique identity barcodes in a combinatorial manner on surfaces, matrices, biomolecules, molecular libraries or biological samples or materials (*e.g*., vesicles, cells, tissues, organoids, droplets, liposomes, small molecules, beads) or to encode unique molecular identities to spatial positions, biomolecules or biological samples or materials. This can be further combined with early approaches such as split-and-pool synthesis or split-pool synthesis where addition of each guide strand is performed following the split of the target population and prior to pooling. See for example, Brenner et al. (2000) Proc. Natl. Acad. Sci. USA 97:1665 and US20160251697A1.

*Constructing spatial barcodes.* The cross-junction synthesis can be used to create barcodes in a combinatorial manner at specified positions or specified targets on surfaces. Non-limiting examples of targets on surfaces include biomolecules, biological materials, and the like. The biological material can be selected from tissues, tissue sections, engineered tissues, cells, patient derived cells, primary cells, organoids, extracellular matrix, 3D biological organs, dissociated cells, live cells, fixed cells, vesicles, droplets, liposomes, etc.

*Strand re-routing.* Because cross-junction synthesis can be used to 're-route' a polymerase to a new backbone sequence, it may be used for gene and sequence editing with synthesis re-routing. This can be done by binding a strand with the new desired sequence in front of the old sequence so that the polymerase copies across a junction onto the new sequence.

In addition to the applications provided above, several types of nucleic acid synthesis can be employed using the compositions and methods of the invention. Synthesis can include but is not limited to single-stranded DNA synthesis, double-stranded DNA synthesis, mutagenesis, insertions, deletions, homologous recombination, parallel/multiplexed synthesis, proximity and molecular distance measurements.

### Selected Definitions

For convenience, the meaning of some terms and phrases used in the specification, examples, and appended claims, are provided below. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments of the aspects provided herein, and are not intended to limit the claimed invention, because the scope of the invention is limited only by the claims. Further, unless otherwise required by context, singular terms shall include pluralities and plural terms shall include the singular.

Definitions of common terms in immunology and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737).

As used herein, "nucleic acid" means DNA, RNA, single-stranded, double-stranded, or more highly aggregated hybridization motifs, and any chemical modifications thereof. For example, a nucleic acid can encompass double- or triple-stranded nucleic acids, as well as single-stranded molecules. In double- or triple-stranded nucleic acids, the nucleic acid strands need not be coextensive (*i.e.,* a double-stranded nucleic acid need not be double-stranded along the entire length of both strands). The term nucleic acid also encompasses any chemical modification thereof, such as by methylation and/or by capping. Nucleic acid modifications can include addition of chemical groups that incorporate additional charge, polarizability, hydrogen bonding, electrostatic interaction, and functionality to the individual nucleic acid bases or to the nucleic acid as a whole. Such modifications may include base modifications such as 2' -position sugar modifications, 5-position pyrimidine modifications, 8-position purine modifications, modifications at cytosine exocyclic amines, substitutions of 5-bromo-uracil, backbone modifications, unusual base pairing combinations such as the isobases isocytidine and isoguanidine, and the like. The nucleic acid(s) can be derived from a completely chemical synthesis process, such as a solid phase-mediated chemical synthesis, from a biological source, such as through isolation from any species that produces nucleic acid, or from processes that involve the manipulation of nucleic acids by molecular biology tools, such as DNA replication, PCR amplification, reverse transcription, or from a combination of those processes.

As used herein, the terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, intergenic DNA, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), small nucleolar RNA, ribozymes, complementary DNA (cDNA), which is a DNA representation of mRNA, usually obtained by reverse transcription of messenger RNA (mRNA) or by amplification; DNA molecules produced synthetically or by amplification, genomic DNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by nonnucleotide components. A polynucleotide may be further modified after polymerization, such as by conjugation with a labeling component.

The term "statistically significant" or "significantly" refers to statistical significance and generally means a two standard deviation (2SD) or greater difference.

As used herein the term "comprising" or "comprises" is used in reference to compositions, methods, and respective component(s) thereof, that are essential to the method or composition, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the invention.

The singular terms "a," "an," and "the" include plural referents unless context clearly indicates otherwise. Similarly, the word "or" is intended to include "and" unless the context clearly indicates otherwise. Although methods and materials similar or equivalent to those provided herein can be used in the practice or testing of this disclosure, suitable methods and materials are described below. The abbreviation, "*e.g*." is derived from the Latin *exempli gratia,* and is used herein to indicate a non-limiting example. Thus, the abbreviation "*e.g*." is synonymous with the term "for example."

Further, unless otherwise required by context, singular terms shall include pluralities and plural terms shall include the singular.

Other than in the operating examples, or where otherwise indicated, all numbers expressing quantities of ingredients or reaction conditions used herein should be understood as modified in all instances by the term "about." The term "about" when used in connection with percentages can mean ±1%.

The term "substantially identical" means two or more nucleotide sequences have at least 65%, 70%, 80%, 85%, 90%, 95%, or 97% identical nucleotides. In some embodiments, "substantially identical" means two or more nucleotide sequences have the same identical nucleotides.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions may include: 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs includes, but not limited to, G:U Wobble or Hoogsteen base pairing.

As used herein, the terms "substrate" or "substrate surface" are used interchangeably to describe a structure upon which one or more nucleic acid templates, guide strands, or portions thereof as provided herein can be displayed or in contact with for contact with additional nucleic acids and/or labels. The nucleic acid template, guide strand, and barcode strands provided herein can be immobilized, conjugated to, or linked to the substrate surface.

As used herein, the term "conjugated to" encompasses association of a nucleic acid with a substrate surface, a phase-changing agent or a member of an affinity pair by covalent bonding, including but not limited to cross-linking via a cross-linking agent, or by a strong non-covalent interaction that is maintained under conditions in which the conjugate is to be used.

As used herein, the term "hybridize" refers to the phenomenon of a single-stranded nucleic acid or region thereof forming hydrogen-bonded base pair interactions with either another single stranded nucleic acid or region thereof (intermolecular hybridization) or with another single-stranded region of the same nucleic acid (intramolecular hybridization). Hybridization is governed by the base sequences involved, with complementary nucleobases forming hydrogen bonds, and the stability of any hybrid being determined by the identity of the base pairs (e.g., G:C base pairs being stronger than A:T base pairs) and the number of contiguous base pairs, with longer stretches of complementary bases forming more stable hybrids.

As used herein, the term "spatial information" is any information, coordinates, markers in a biological tissue or matrix, that can be stored in the barcode. The spatial information can inform one of skill in the art where on the substrate a particular marker, barcode, or pattern is located. For example, spatial information may be useful in creating an image or QR code with the nucleic acid barcodes. Spatial information can also be useful in the detection of a specific nucleic acid target.

It should be understood that this disclosure is not limited to the particular methodology, protocols, and reagents, etc., provided herein and as such may vary. The terminology used herein is for the purpose of describing particular embodiments only, and is not intended to limit the scope of the present disclosure, which is defined solely by the claims. The invention is further illustrated by the following example, which should not be construed as further limiting.

### EXAMPLES

### EXAMPLE 1: A METHOD FOR SYNTHESIZING STRANDS ACROSS NUCLEIC ACID JUNCTIONS

The ability to synthesize arbitrary sequences of nucleic acids, in particular DNA, has revolutionized how scientists can study and engineer biology. Whole genomes can now be sequenced effectively (Schwarze *et al.,* 2018) but the technology to synthesize genomes has lagged behind. Synthetic single-stranded sequences (`oligos') are typically synthesized chemically through cyclic coupling steps (Caruthers *et al.,* 1987). However, oligos are typically not chemically synthesized past two hundred bases, due to the limitations on chemical coupling efficiency. Therefore, synthesis methods have been developed to assemble larger fragments of single-stranded and double-stranded sequences, including enzymatic assembly using sequential or simultaneous combination of multiple enzyme activities (such as restriction enzyme digestion and ligation, or isothermal Gibson assembly (Gibson, 2011; Gibson *et al.,* 2009) which combines 5' exonuclease, the 3' extension activity of a DNA polymerase and DNA ligase activity), non-enzymatic twin primer assembly (Liang *et al.,* 2017), and chemical assembly (such as click DNA assembly (Kukwikila *et al.,* 2017)). While these can be effective at producing longer sequences of single-stranded and double-stranded DNA, sequences of hundreds of thousands of bases must be further assembled from shorter fragments in vivo using yeast vectors (Hutchison *et al.,* 2016). A new simple and robust synthesis method capable of generating long sequences of DNA could have important enabling applications in molecular biology, genome engineering, nanotechnology, and polymer-based data storage. A method for synthesizing strands of nucleic acid across different nucleic acid backbones hybridized together using a strand displacing polymerase is described herein. This method for synthesizing strands across nucleic acid junctions can effectively decouple the assembly step of template oligos from the synthesis step. In this way it is possible to sequentially perform robust annealing of template oligos, that can hybridize in predictable ways close to thermodynamic optimum, and follow up with polymerization to create the new transcript. This method has the potential to scale to very long and arbitrary sequences. Moreover, the entire assembly and synthesis process can happen isothermally, including at room temperature, which enables a generalized workflow for synthesis of long arbitrary sequences under relatively mild conditions.

The fundamental strategy for cross-junction synthesis is depicted in **FIG. 1A-1B****.** Shown are two template oligos that have been hybridized together to form a junction, and a primer that has bound in front of that junction on the first template strand (left). A strand displacing polymerase is used to copy the x domain until it reaches a stopper (shown in black). Afterwards, the new and old x domains compete in a random walk branch migration process (Lee *et al.,* 1970). Ultimately, the new x domain can bind to the exposed x* domain on the second template strand (right), thus successfully crossing the junction. Polymerization can then continue on the second template strand, copying along a new backbone.

By hybridizing multiple template oligos together, cross-junction synthesis reactions can be cascaded to form longer sequences, as depicted in **FIG. 2****.** Each junction between template strands shows the same domain motifs, whereby the strand domains copied on the 3' end of the growing strand before reaching a stopper (**b, c, d, e**) can reach across the junction and bind to the exposed complementary sequence (**b*, c*, d*, e***) on the next template strand. Arbitrary sequences can be added in the template regions between the motif domains (shown in gray) to enable longer sequences to be assembled.

This method is able to concatenate arbitrary prescribed sequences, and only requires the fundamental domain constraints depicted in **FIG. 1A-1B**.

It is also suitable for synthesizing specific numbers of repeated sequences through the use of programmable unique hybridization domains that fix the length of the template concatemer, which has been a long-standing challenge. By harnessing the specificity of DNA self-assembly, unique binding sequences promote assemblies of prescribed length, even if the domains being synthesized are identical (**FIG. 3**).

A strategy for assembling cross-junctions with light will utilize a photoreactive inter-strand crosslinking base, which can serve the dual purpose of linking two cross-junction strands together and acting as a stopper for polymerase synthesis (**FIG. 4A-4C**). Crossjunctions can be assembled iteratively through cycles of hybridization, crosslinking and washing. Cross-junctions can also be assembled and spatially addressed on a surface, and subsequent synthesis steps can still be performed on the surface.

Assembling a library of orthogonal cross-junction arrays on a surface can enable multiplexed synthesis of orthogonal, and arbitrary length DNA of prescribed sequences.

In the current implementation, a 3-Cyanovinylcarbazole (CNVK) (Vieregg *et al.,* 2013) base modification and a UV light source to crosslink DNA junctions together was used. Strand design can utilize a set of two or more hybridization domains (1 and 1*, 2 and 2* in current implementation), an internal barcode sequence (b1, b2, etc....) that will serve as the growing sequence chain, and the CNVK base crosslinker (**FIG. 4A****,** cyan circle).

### Experimental validation

Cascaded cross-junction synthesis, as well as the stepwise assembly of template concatemers on a surface using photoreactive crosslinking chemistry (see above) followed by cross-junction synthesis, were validated experimentally.

Synthesis cascades ranging from one to eight-junction synthesis as depicted in **FIGS. 5-12** were designed utilizing random three-letter (A, T, and C) 7nt 'flap' primers (sequences **b, d, f, h, j, l,** and **n**), three consecutive G's as a stopper (with dGTP excluded from dNTP mix), random four-letter 9nt secondary hybridization domains (**r0, r1,** ..., **r7**), and 5nt three-letter (A, T, and C) intercalating domains (**c, e, g, i, k, m, o, q)**. Template concatemers were formed by combining strands together at 1uM concentration in 1x PBS and annealing from 80C to 20C over one hour (reducing one degree per minute). Concatemers were then introduced to the synthesis reaction at a final concentration of 50 nM in 0.95xPBS with 10 mM MgSO4 with the **a** primer at 40nM, a dGTP cleanup hairpin at 1uM, 600 uM dATP/dCTP/dTTP mix, and 800 units/ml of Bst Large Fragment polymerase. Reactions were incubated at room temperature for 15 minutes before the primer was added and incubation continued for a further 30 minutes. 2uL of 6mM dGTP was added to 20uL reactions and incubated for a further 5 minutes to complete the copying of the **For** primer sequence. Reactions were then heat inactivated for 20 minutes at 80C before being diluted 1000x (final) into a Taq 1X PCR with 200 nM **For** and **rev** primers. A qPCR machine was used to track amplification in real-time using the Sybr Green I intercalating time (**FIG. 13A**), and PCR products were run on a PAGE denaturing gel (**FIG. 13B**). The longest, eight-junction synthesis product was further validated using Sanger sequencing (**FIG. 13C**).

The light-directed concatemer formation (see above) was also experimentally validated. The reactions performed are depicted in **FIGS. 14-16****,** and the result is shown in **FIG. 17****.** After probe sequences were bound to glass slides, template strands were hybridized at 1uM concentration in 1x PBS for 5 minutes, bound strands were crosslinked with UV light for ~3 seconds, and then several wash steps were performed before the next hybridization step with 1x PBS to remove unbound strands. All of these steps took place at room temperature. After template formation, cross-junction synthesis was performed with 40nM primer at room temperature, and the result was heat inactivated at 80C for 20 minutes before being diluted into a PCR solution and amplified to form the double stranded product. A PAGE denaturing gel was run to show the products for three different template sizes (**FIG. 17**).

### Discussion

The novel ability to program synthesis across nucleic acid junctions enables a new paradigm of synthesis that is no longer limited to copying along a single backbone. The formation of the template concatemer (the assembly step) can be performed under favorable conditions, such as through annealing that can help to improve the specificity of the formed hybridization interactions. The primer strand may be included in the formation step or hybridized later, and cross-junction synthesis proceeds with a strand displacing polymerase to produce sequence records of the template concatemers. As shown above, arbitrary programmable sequences can be assembled in this way to produce long single-stranded sequences. Single-stranded sequences can either directly be used or amplified (such as with PCR) to form single-stranded or double-stranded products. This capability opens up a number of exciting applications ranging from the generation of combinatorial libraries of sequences to *in situ* synthesis.

### References

1. Caruthers, M.H., Barone, A.D., Beaucage, S.L., Dodds, D.R., Fisher, E.F., McBride, L.J., Matteucci, M., Stabinsky, Z., and Tang, J.-Y. (1987). [15] Chemical synthesis of deoxyoligonucleotides by the phosphoramidite method. In Methods in Enzymology, (Academic Press), pp. 287-313.
2. Gibson, D.G. (2011). Chapter fifteen - Enzymatic Assembly of Overlapping DNA Fragments. In Methods in Enzymology, C. Voigt, ed. (Academic Press), pp. 349-361.
3. Gibson, D.G., Young, L., Chuang, R.-Y., Venter, J.C., Hutchison, C.A., 3rd, and Smith, H.O. (2009). Enzymatic assembly of DNA molecules up to several hundred kilobases. Nat. Methods 6, 343-345.
4. Hutchison, C.A., 3rd, Chuang, R.-Y., Noskov, V.N., Assad-Garcia, N., Deerinck, T.J., Ellisman, M.H., Gill, J., Kannan, K., Karas, B.J., Ma, L., et al. (2016). Design and synthesis of a minimal bacterial genome. Science 351, aad6253.
5. Kukwikila, M., Gale, N., El-Sagheer, A.H., Brown, T., and Tavassoli, A. (2017). Assembly of a biocompatible triazole-linked gene by one-pot click-DNA ligation. Nat. Chem. 9, 1089-1098.
6. Lee, C.S., Davis, R.W., and Davidson, N. (1970). A physical study by electron microscopy of the terminally repetitious, circularly permuted DNA from the coliphage particles of Escherichia coli 15. J. Mol. Biol. 48, 1-22.
7. Liang, J., Liu, Z., Low, X.Z., Ang, E.L., and Zhao, H. (2017). Twin-primer non-enzymatic DNA assembly: an efficient and accurate multi-part DNA assembly method. Nucleic Acids Res. 45, e94.
8. Schwarze, K., Buchanan, J., Taylor, J.C., and Wordsworth, S. (2018). Are whole-exome and whole-genome sequencing approaches cost-effective? A systematic review of the literature. Genet. Med. 20, 1122-1130.
9. Vieregg, J.R., Nelson, H.M., Stoltz, B.M., and Pierce, N.A. (2013). Selective nucleic acid capture with shielded covalent probes. J. Am. Chem. Soc. 13, 9691-9699.

### EXAMPLE 2: NUCLEIC ACID SYNTHESIS ACROSS MULTIPLE JUNCTIONS

In addition to the methods described above, synthesis across 8-junctions was performed using a fluorophore-labeled primer following the process outlined in **FIG. 19A****.** Template strands (also called guide strands) were annealed (1µM each strand in 1×PBS, from 80°C to 20°C over 1 hour) and diluted into various reaction buffer conditions at two time points (10 and 20 minutes). The template sequences utilized G-C pairs to stop polymerization, and reactions were pre-incubated for 5 minutes with a hairpin. The hairpin can sequester any extraneous dGTP in a mix of dATP, dTTP, and dCTP before the fluorophore-labeled primer can be added (at 40nM final, with each template strand at 100nM). Reactions were then incubated for two minutes at the depicted temperature for 10 or 20 minutes before dGTP was added. This step was followed by heat inactivation at 80°C for 20 minutes. Reactions were run on a 15% TBE-Urea PAGE denaturing gel to determine the effect on synthesis efficiency (**FIG. 19B**). The two time points (10 and 20 minutes) allowed for monitoring of the reaction process over time. The more bands that appeared on the gel indicated that synthesis had occurred across a higher number of junctions. Lanes 1 and 7 were found to have the highest efficiency for nucleic acid synthesis using the method shown in FIG. 19A (**FIG. 19B**).

Several salt conditions were also tested with a 5-junction cross-synthesis reaction. In this example of the methods described herein, longer binding domains between template strands were used. The primer and template strands were annealed together at increasing concentrations in 1×PBS, from 80°C to 20°C over a 1-hour incubation period. A total of 48 reaction conditions (8 salt conditions × 3 polymerase concentrations × two time points) were tested with incubations at 37°C followed by incubation with dGTP and heat inactivation at 80°C for 20 minutes. Efficiency was evaluated based on the Cq value from amplification of the full reaction products (**FIG. 20A-20B**).

In another example, cross-junction synthesis was performed by continuously adding one template strand at a time rather than pre-assembly of the template strands as described above (see schematic in **FIG. 21A**). In continuous nucleic acid synthesis, initially the full reaction conditions for a 5-junction cross-synthesis reaction minus any oligos are pre-incubated before the primer is introduced. After every 2 minutes of incubation at 37°C, a new template strand was added, in the order of synthesis. To ensure nucleic acid synthesis was occurring while each template strand was continuously added, a separate strand (terminal barcode strand) was added. The terminal barcode strand used in this example comprised (i) one or more substantially identical binding domains to the third template strand; and (ii) a different barcode. The barcode is not complementary to the template strand. The terminal barcode strand was added at the end of the synthesis process. Finally, the reaction mixture was incubated with dGTP and was heat inactivated. The complete product was amplified by PCR and Sanger sequencing was performed (**FIG. 21B**). The sequencing results show the nucleic acid sequences without interference of the barcode from the last terminal barcode strand (**FIG. 21B****,** bottom).

## Claims

1. A nucleic acid template comprising:
(a) a first guide strand comprising in 3' to 5' direction a first synthesis region, a second synthesis region, a first junction domain, and a first blocking region; and
(b) a second guide strand comprising in 3' to 5' direction a second blocking region, a second junction domain and a third synthesis region,
wherein the first junction domain comprises a nucleotide sequence substantially identical to a nucleotide sequence of the third synthesis region, and the first junction domain and second junction domain are substantially complementary to each other and form a double-stranded region, and
wherein the first blocking region and the second blocking region together form a first blocking domain that blocks strand displacement activity of a polymerase.

2. The nucleic acid template of claim 1, wherein:
(a) the first and the second blocking regions are covalently linked to each other; and/or
(b) the first or the second blocking region comprises a cross-linking segment with the other blocking region to form the first blocking domain, optionally wherein the cross-linking segment comprises 3-cyanovinylcarbozole.

3. The nucleic acid template of claim 1, wherein the second guide strand further comprises a fourth synthesis region at the 5' end of the third synthesis region;
optionally wherein the second guide strand further comprises at its 5' end in 3' to 5' direction a third junction domain and a third blocking region, and the nucleic acid template further comprises a third guide strand comprising in 3' to 5' direction a fourth blocking region, a fourth junction domain and a fifth synthesis region,
wherein the third junction domain comprises a nucleotide sequence substantially identical to a nucleotide sequence of the fifth synthesis region,
wherein the third junction domain and fourth junction domain are substantially complementary to each other and form a double-stranded region, and
wherein the third blocking region and the fourth blocking region together form a second blocking domain that blocks strand displacement activity of a polymerase.

4. The nucleic acid template of claim 3, wherein:
(a) the third and the fourth blocking regions are covalently linked to each other; and/or
(b) the third or the fourth blocking region comprises a cross-linking segment with the other blocking region to form the second blocking domain, optionally wherein the cross-linking segment comprises 3-cyanovinylcarbozole.

5. The nucleic acid template of claim 3, wherein the third guide strand further comprises a sixth synthesis region at the 5' end of the fifth synthesis region;
optionally wherein the third guide strand further comprises at its 5' end in 3' to 5' direction a fifth junction domain and a fifth blocking region, and the nucleic acid template further comprises a fourth guide strand comprising in 3' to 5' direction a sixth blocking region, a sixth junction domain and a seventh synthesis region,
wherein the fifth junction domain comprises a nucleotide sequence substantially identical to a nucleotide sequence of the seventh synthesis region, and the fifth junction domain and sixth junction domain are substantially complementary to each other and form a double-stranded region, and
wherein the fifth blocking region and the sixth blocking region together form a third blocking domain that blocks strand displacement activity of a polymerase.

6. The nucleic acid template of claim 5, wherein:
(a) the fifth and sixth blocking regions are covalently linked to each other; and/or
(b) the fifth or the sixth blocking region comprises a cross-linking segment with the other blocking region to form the third blocking domain,
optionally wherein the cross-linking segment comprises 3-cyanovinylcarbozole.

7. The nucleic acid template of claim 5, wherein the fourth guide strand further comprises an eighth synthesis region at the 5' end of the seventh synthesis region;
optionally wherein the fourth guide strand further comprises at its 5' end in 3' to 5' direction a seventh junction domain and a seventh blocking region, and the nucleic acid template further comprises a fifth guide strand comprising in 3' to 5' direction an eighth blocking region, an eighth junction domain and a ninth synthesis region,
wherein the seventh junction domain comprises a nucleotide sequence substantially identical to a nucleotide sequence of the ninth synthesis region, and the seventh junction domain and eighth junction domain are substantially complementary to each other and form a double-stranded region, and
wherein the seventh blocking region and the eighth blocking region together form a fourth blocking domain that blocks strand displacement activity of a polymerase.

8. The nucleic acid template of claim 7, wherein:
(a) the seventh and eighth blocking regions are covalently linked to each other; and/or
(b) the seventh or the eighth blocking region comprises a cross-linking segment with the other blocking region to form the fourth blocking domain, optionally wherein the cross-linking segment comprises 3- cyanovinylcarbozole.

9. The nucleic acid template of any one of the preceding claims, wherein:
(a) the cross-linking segment comprises 3-cyanovinylcarbozole;
(b) one or more regions utilize a 3-letter code; or
(c) at least one of the first, second, third or fourth blocking domain comprises a double stranded region or a poly monomer stretch.

10. The nucleic acid template of claim 1, wherein the nucleic acid template or at least one of the first, second, third or fourth blocking domain comprises a nucleic acid modification;
optionally wherein the nucleic acid modification is a modified nucleobase or a fluorophore on the 3' and/or 5'-end.

11. The nucleic acid template of claim 1, wherein the nucleic acid template:
(a) is conjugated to a solid support;
(b) further comprises a primer annealed or hybridized to the one of the guide strands; or
(c) further comprises a primer annealed or hybridized to the first guide strand.

12. A method for forming a nucleic acid template, the method comprising annealing or hybridizing a first guide strand and a second guide strand, wherein:
(a) the first guide strand comprises in 3' to 5' direction a first synthesis region, a second synthesis region, a first junction domain, and a first blocking region; and
(b) the second guide strand comprises in 3' to 5' direction a second blocking region, a second junction domain and a third synthesis region,
wherein the first junction domain comprises a nucleotide sequence substantially identical to a nucleotide sequence of the third synthesis region, and the first junction domain and second junction domain are substantially complementary to each other and form a double-stranded region, and
wherein the first blocking region and the second blocking region together form a blocking domain that blocks strand displacement activity of a polymerase.

13. A method for synthesizing a nucleic acid sequence, the method comprising:
(a) providing or obtaining a nucleic acid template of any one of claims 1-11;
(b) annealing or hybridizing a primer, if not already annealed, to the nucleic acid template; and
(c) extending a nucleotide sequence using a DNA polymerase having strand displacement activity from the 3'-terminus of the primer;
optionally wherein the method further comprises amplifying the nucleic acid sequence or further comprises sequencing the synthesized nucleic acid sequence.

14. A method for synthesizing a nucleic acid sequence, the method comprising:
(a) annealing or hybridizing a primer, if not already annealed, to a first guide strand, wherein the first guide strand comprises in a 3' to 5' direction a first synthesis region, a second synthesis region, a junction domain and a blocking region; and
(b) extending a nucleotide sequence using a DNA polymerase having strand displacement activity from the 3'-terminus of the primer;
(c) adding a second guide strand to the synthesis reaction, where the second guide strand comprises in a 3' to 5' direction a blocking region, a junction domain, a first synthesis region, a second synthesis region, a second junction domain, and a second blocking region, wherein the first blocking domain of the second guide strand and the blocking domain of the first guide strand together form a blocking domain that blocks strand displacement activity of a polymerase, the first junction domain of the second guide strand comprises a nucleotide sequence substantially complementary to a nucleic acid sequence of the junction domain of the first guide strand to form a double-stranded region, and the first synthesis region of the second guide strand comprises a nucleotide sequence substantially identical to a nucleic acid sequence of the junction domain of the first guide strand;
(d) optionally, cross-linking the first blocking domain of the second guide strand and the blocking domain of the first guide strand;
(e) further extending the nucleotide sequence;
(f) optionally, adding additional new guide strands to the synthesis reaction and further extending the nucleotide sequence prior to addition of each additional new guide strand, wherein each additional guide strand comprises in a 3' to 5' direction a blocking region, a junction domain, a first synthesis region, a second synthesis region, a second junction domain, and a second blocking region, wherein the first blocking region of the new guide strand and the second blocking region of the last guide strand added to the synthesis reaction together form a blocking domain that blocks strand displacement activity of a polymerase; the first junction domain of the new guide strand comprises a nucleotide sequence substantially complementary to a nucleic acid sequence of the second junction domain of the last guide strand added to the synthesis reaction to form a double-stranded region; and the first synthesis region of the new guide strand comprises a nucleotide sequence substantially identical to a nucleic acid sequence of the second junction domain of the last guide strand added to the synthesis reaction;
(g) optionally, cross-linking the first blocking region of the new guide strand and the second blocking region of the last guide strand added to the synthesis reaction prior to further extending the nucleotide sequence;
optionally further comprising:
(h) adding a terminal guide strand to synthesis reaction, where the terminal guide strand comprises in a 3' to 5' direction a blocking region, a junction domain, a first synthesis region, a second synthesis region, a second junction domain, and a second blocking region, wherein the first blocking region of the terminal guide strand and the second blocking region of the last guide strand added to the synthesis reaction together form a blocking domain that blocks strand displacement activity of a polymerase; the first junction domain of the terminal guide strand comprises a nucleotide sequence substantially complementary to a nucleic acid sequence of the second junction domain of the last guide strand added to the synthesis reaction to form a double-stranded region; and the first synthesis region of the terminal guide strand comprises a nucleotide sequence substantially identical to a nucleic acid sequence of the second junction domain of the last guide strand added to the synthesis reaction;
(i) optionally, cross-linking the first blocking domain of the terminal guide strand and the blocking domain of the last guide strand added to the reaction; and
(j) further extending the nucleotide sequence;
optionally wherein the method further comprises amplifying the nucleic acid sequence or further comprises sequencing the synthesized nucleic acid sequence.

15. Use of a nucleic acid template of any one of claims 1-11 for:
(a) creating combinatorial barcodes to assign unique identities to a target population, wherein the target population is surface positions/partitions, matrices, biomolecules, molecular libraries or biological material, optionally wherein the biological material is vesicles, cells, tissues, organoids, droplets, liposomes, small molecules, or beads, and optionally the use comprising splitting the target population prior to addition of a guide strand and pooling the split target population after addition of the guide strand;
(b) creating combinatorial barcodes to assign spatial position identifiers to a target, wherein the target is a surface, biomolecule, or biological material, optionally wherein the biological material is biomolecules, cells, tissues, organs, organoids, vesicles, liposomes, or droplets;
(c) determining the proximity of biomolecules and creating records indicative of distance information between labeled biomolecules; or
(d) generating a nucleic acid sequence library.

## Patentansprüche

1. Nukleinsäurematrize, umfassend:
(a) einen ersten Führungsstrang, der in 3'-nach-5'-Richtung einen ersten Synthesebereich, einen zweiten Synthesebereich, eine erste Kreuzungsdomäne und einen ersten Blockierbereich umfasst;
(b) einen zweiten Führungsstrang, der in 3'-nach-5'-Richtung einen zweiten Blockierbereich, eine zweite Kreuzungsdomäne und einen dritten Synthesebereich umfasst,
wobei die erste Kreuzungsdomäne eine Nukleotidsequenz umfasst, die im Wesentlichen identisch mit einer Nukleotidsequenz des dritten Synthesebereichs ist, und die erste Kreuzungsdomäne und die zweite Kreuzungsdomäne im Wesentlichen komplementär zueinander sind und einen doppelsträngigen Bereich bilden, und wobei der erste Blockierbereich und der zweite Blockierbereich zusammen eine erste Blockierdomäne bilden, die Strangverdrängungsaktivität einer Polymerase blockiert.

2. Nukleinsäurematrize nach Anspruch 1, wobei:
(a) der erste und der zweite Blockierbereich kovalent miteinander verbunden sind; und/oder
(b) der erste oder der zweite Blockierbereich ein Vernetzungssegment mit dem anderen Blockierbereich umfasst, um die erste Blockierdomäne zu bilden, wobei das Vernetzungssegment optional 3-Cyanovinylcarbozol umfasst.

3. Nukleinsäurematrize nach Anspruch 1, wobei der zweite Führungsstrang ferner einen vierten Synthesebereich am 5'-Ende des dritten Synthesebereichs umfasst;
wobei der zweite Führungsstrang an seinem 5'-Ende in 3'-nach-5'-Richtung optional ferner eine dritte Kreuzungsdomäne und einen dritten Blockierbereich umfasst und die Nukleinsäurematrize ferner einen dritten Führungsstrang umfasst, der in 3'-nach-5'-Richtung einen vierten Blockierbereich, eine vierte Kreuzungsdomäne und einen fünften Synthesebereich umfasst,
wobei die dritte Kreuzungsdomäne eine Nukleotidsequenz umfasst, die im Wesentlichen identisch mit einer Nukleotidsequenz des fünften Synthesebereichs ist,
wobei die dritte Kreuzungsdomäne und die vierte Kreuzungsdomäne im Wesentlichen komplementär zueinander sind und einen doppelsträngigen Bereich bilden, und
wobei der dritte Blockierbereich und der vierte Blockierbereich zusammen eine zweite Blockierdomäne bilden, die Strangverdrängungsaktivität einer Polymerase blockiert.

4. Nukleinsäurematrize nach Anspruch 3, wobei:
(a) der dritte und der vierte Blockierbereich kovalent aneinander gebunden sind; und/oder
(b) der dritte oder der vierte Blockierbereich ein Vernetzungssegment mit dem anderen Blockierbereich umfasst, um die zweite Blockierdomäne zu bilden, wobei das Vernetzungssegment optional 3-Cyanovinylcarbozol umfasst.

5. Nukleinsäurematrize nach Anspruch 3, wobei der dritte Führungsstrang ferner einen sechsten Synthesebereich am 5'-Ende des fünften Synthesebereichs umfasst;
wobei der dritte Führungsstrang an seinem 5'-Ende in 3'-nach-5'-Richtung optional ferner eine fünfte Kreuzungsdomäne und einen fünften Blockierbereich umfasst und die Nukleinsäurematrize ferner einen vierten Führungsstrang umfasst, der in 3'-nach-5'-Richtung einen sechsten Blockierbereich, eine sechste Kreuzungsdomäne und einen siebten Synthesebereich umfasst,
wobei die fünfte Kreuzungsdomäne eine Nukleotidsequenz umfasst, die im Wesentlichen identisch mit einer Nukleotidsequenz des siebten Synthesebereichs ist, und die fünfte Kreuzungsdomäne und die sechste Kreuzungsdomäne im Wesentlichen komplementär zueinander sind und einen doppelsträngigen Bereich bilden, und wobei der fünfte Blockierbereich und der sechste Blockierbereich zusammen eine dritte Blockierdomäne bilden, die Strangverdrängungsaktivität einer Polymerase blockiert.

6. Nukleinsäurematrize nach Anspruch 5, wobei:
(a) der fünfte und der sechste Blockierbereich kovalent aneinander gebunden sind; und/oder
(b) der fünfte oder der sechste Blockierbereich ein Vernetzungssegment mit dem anderen Blockierbereich umfasst, um die dritte Bockierdomäne zu bilden,
wobei das Vernetzungssegment optional 3-Cyanvinylcarbozol umfasst.

7. Nukleinsäurematrize nach Anspruch 5, wobei der vierte Führungsstrang ferner einen achten Synthesebereich am 5'-Ende des siebten Synthesebereichs umfasst;
wobei der vierte Führungsstrang an seinem 5'-Ende in 3'-nach-5'-Richtung optional ferner eine siebte Kreuzungsdomäne und einen siebten Blockierbereich umfasst und die Nukleinsäurematrize ferner einen fünften Führungsstrang umfasst, der in 3'-nach-5'-Richtung einen achten Blockierbereich, eine achte Kreuzungsdomäne und einen neunten Synthesebereich umfasst,
wobei die siebte Kreuzungsdomäne eine Nukleotidsequenz umfasst, die im Wesentlichen identisch mit einer Nukleotidsequenz des neunten Synthesebereichs ist, und die siebte Kreuzungsdomäne und die achte Kreuzungsdomäne im Wesentlichen komplementär zueinander sind und einen doppelsträngigen Bereich bilden, und wobei der siebte Blockierbereich und der achte Blockierbereich zusammen eine vierte Blockierdomäne bilden, die Strangverdrängungsaktivität einer Polymerase blockiert.

8. Nukleinsäurematrize nach Anspruch 7, wobei:
(a) der siebte und der achte Blockierbereich kovalent aneinander gebunden sind; und/oder
(b) der siebte oder der achte Blockierbereich ein Vernetzungssegment mit dem anderen Blockierbereich umfasst, um die vierte Blockierdomäne zu bilden, wobei das Vernetzungssegment optional 3-Cyanovinylcarbozol umfasst.

9. Nukleinsäurematrize nach einem der vorhergehenden Ansprüche, wobei:
(a) das Vernetzungssegment 3-Cyanvinylcarbozol umfasst;
(b) ein oder mehrere Bereiche einen 3-Buchstaben-Code verwenden; oder
(c) mindestens eine der ersten, der zweiten, der dritten oder der vierten Blockierdomäne einen doppelsträngigen Bereich oder eine Polymonomerstrecke umfasst.

10. Nukleinsäurematrize nach Anspruch 1, wobei die Nukleinsäurematrize oder mindestens eine der ersten, der zweiten, der dritten oder der vierten Blockierdomäne eine Nukleinsäuremodifikation umfasst;
wobei die Nukleinsäuremodifikation optional eine modifizierte Nukleobase oder ein Fluorophor am 3'- und/oder 5'-Ende ist.

11. Nukleinsäurematrize nach Anspruch 1, wobei die Nukleinsäurematrize:
(a) an einen festen Träger konjugiert ist;
(b) ferner einen mit dem einen der Führungsstränge annealten oder hybridisierten Primer umfasst; oder
(c) ferner einen mit dem ersten Führungsstrang annealten oder hybridisierten Primer umfasst.

12. Verfahren zum Bilden einer Nukleinsäurematrize, wobei das Verfahren Annealen oder Hybridisieren eines ersten Führungsstrangs und eines zweiten Führungsstrangs umfasst, wobei:
(a) der erste Führungsstrang in 3'-nach-5'-Richtung einen ersten Synthesebereich, einen zweiten Synthesebereich, eine erste Kreuzungsdomäne und einen ersten Blockierbereich umfasst;
(b) der zweite Führungsstrang in 3'-nach-5'-Richtung einen zweiten Blockierbereich, eine zweite Kreuzungsdomäne und einen dritten Synthesebereich umfasst,
wobei die erste Kreuzungsdomäne eine Nukleotidsequenz umfasst, die im Wesentlichen identisch mit einer Nukleotidsequenz des dritten Synthesebereichs ist, und die erste Kreuzungsdomäne und die zweite Kreuzungsdomäne im Wesentlichen komplementär zueinander sind und einen doppelsträngigen Bereich bilden, und wobei der erste Blockierbereich und der zweite Blockierbereich zusammen eine Blockierdomäne bilden, die Strangverdrängungsaktivität einer Polymerase blockiert.

13. Verfahren zum Synthesieren einer Nukleinsäuresequenz, wobei das Verfahren umfasst:
(a) Bereitstellen oder Erhalten einer Nukleinsäurematrize nach einem der Ansprüche 1-11;
(b) Annealen oder Hybridisieren eines Primers, falls er nicht bereits annealt wurde, mit der Nukleinsäurematrize; und
(c) Verlängern einer Nukleotidsequenz unter Verwendung einer DNA-Polymerase, die Strangverschiebungsaktivität aufweist, vom 3'-Terminus des Primers;
wobei das Verfahren ferner optional Amplifizieren der Nukleinsäuresequenz umfasst oder ferner Sequenzieren der synthetisierten Nukleinsäuresequenz umfasst.

14. Verfahren zum Synthesieren einer Nukleinsäuresequenz, wobei das Verfahren umfasst:
(a) Annealen oder Hybridisieren eines Primers, falls er nicht bereits annealt wurde, mit einem ersten Führungsstrang, wobei der erste Führungsstrang in 3'-nach-5'-Richtung einen ersten Synthesebereich, einen zweiten Synthesebereich, eine Kreuzungsdomäne und einen Blockierbereich umfasst; und
(b) Verlängern einer Nukleotidsequenz unter Verwendung einer DNA-Polymerase, die Strangverschiebungsaktivität aufweist, vom 3'-Terminus des Primers;
(c) Zusetzen eines zweiten Führungsstrangs zu der Synthesereaktion, wobei der zweite Führungsstrang in einer 3'-nach-5'-Richtung einen Blockierbereich, eine Kreuzungsdomäne, einen ersten Synthesebereich, einen zweiten Synthesebereich, eine zweite Blockierdomäne und einen zweiten Blockierbereich umfasst, wobei die erste Blockierdomäne des zweiten Führungsstrangs und die Blockierdomäne des ersten Führungsstrangs zusammen eine Blockierdomäne bilden, die Strangverschiebungsaktivität einer Polymerase blockiert, die erste Kreuzungsdomäne des zweiten Führungsstrangs eine Nukleotidsequenz umfasst, die im Wesentlichen komplementär zu einer Nukleotidsequenz der Kreuzungsdomäne des ersten Führungsstrangs ist, um einen doppelsträngigen Bereich zu bilden, und der erste Synthesebereich des zweiten Führungsstrangs eine Nukleotidsequenz umfasst, die im Wesentlichen identisch mit einer Nukleotidsequenz der Kreuzungsdomäne des ersten Führungsstrangs ist;
(d) optional Vernetzen des ersten Blockierbereichs des zweiten Führungsstrangs und der Blockierdomäne des ersten Führungsstrangs;
(e) weiteres Verlängern der Nukleotidsequenz;
(f) optional Zusetzen weiterer neuer Führungsstränge zu der Synthesereaktion und weiteres Verlängern der Nukleotidsequenz vor dem Zusetzen jedes weiteren neuen Führungsstrangs, wobei jeder zusätzliche Führungsstrang in 3'-nach-5'-Richtung einen Blockierbereich, eine Kreuzungsdomäne, einen ersten Synthesebereich, einen zweiten Synthesebereich, eine zweite Kreuzungsdomäne und einen zweiten Blockierbereich umfasst, wobei der erste Blockierbereich des neuen Führungsstrangs und der zweite Blockierbereich des letzten der Synthesereaktion zugesetzten Führungsstrangs zusammen eine Blockierdomäne bilden, die Strangverdrängungsaktivität einer Polymerase blockiert; die erste Kreuzungsdomäne des neuen Führungsstrangs eine Nukleotidsequenz umfasst, die im Wesentlichen komplementär zu einer Nukleinsäuresequenz der zweiten Kreuzungsdomäne des letzten der Synthesereaktion zugesetzten Führungsstrangs ist, um einen doppelsträngigen Bereich zu bilden; und der erste Synthesebereich des neuen Führungsstrangs eine Nukleotidsequenz umfasst, die im Wesentlichen identisch mit einer Nukleinsäuresequenz der zweiten Kreuzungsdomäne des letzten der Synthesereaktion zugesetzten Führungsstrangs ist;
(g) optional Vernetzen des ersten Blockierbereichs des neuen Führungsstrangs und des zweiten Blockierbereichs des letzten der Synthesereaktion zugesetzten Führungsstrangs vor einem weiteren Verlängern der Nukleotidsequenz;
optional ferner umfassend:
(h) Zusetzen eines terminalen Führungsstrangs zu der Synthesereaktion, wobei der terminale Führungsstrang in 3'-nach-5'-Richtung einen Blockierbereich, eine Kreuzungsdomäne, einen ersten Synthesebereich, einen zweiten Synthesebereich, eine zweite Kreuzungsdomäne und einen zweiten Blockierbereich umfasst, wobei der erste Blockierbereich des terminalen Führungsstrangs und der zweite Blockierbereich des letzten der Synthesereaktion zugesetzten Führungsstrangs zusammen eine Blockierdomäne bilden, die Strangverdrängungsaktivität einer Polymerase blockiert; die erste Kreuzungsdomäne des terminalen Führungsstrangs eine Nukleotidsequenz umfasst, die im Wesentlichen komplementär zu einer Nukleinsäuresequenz der zweiten Kreuzungsdomäne des letzten der Synthesereaktion zugesetzten Führungsstrangs ist, um einen doppelsträngigen Bereich zu bilden; und der erste Synthesebereich des terminalen Führungsstrangs eine Nukleotidsequenz umfasst, die im Wesentlichen identisch mit einer Nukleinsäuresequenz der zweiten Kreuzungsdomäne des letzten der Synthesereaktion zugesetzten Führungsstrangs ist;
(i) optional Vernetzen der ersten Blockierdomäne des terminalen Führungsstrangs und der Blockierdomäne des letzten der Reaktion zugesetzten Führungsstrangs; und
(e) weiteres Verlängern der Nukleotidsequenz;
wobei das Verfahren optional ferner Amplifizieren der Nukleinsäuresequenz umfasst oder ferner Sequenzieren der synthetisierten Nukleinsäuresequenz umfasst.

15. Verwendung einer Nukleinsäurematrize nach einem der Ansprüche 1-11 zum:
(a) Erstellen kombinatorischer Barcodes, um einer Zielpopulation eindeutige Identitäten zuzuordnen, wobei es sich bei der Zielpopulation um Oberflächenpositionen/-teilbereiche, Matrizen, Biomoleküle, Molekülbibliotheken oder biologisches Material handelt, wobei es sich bei dem biologischen Material optional um Vesikel, Zellen, Gewebe, Organoide, Tröpfchen, Liposomen, niedermolekulare Verbindungen oder Perlen handelt, und die Verwendung optional Aufteilen der Zielpopulation vor Zusatz eines Führungsstrangs und Poolen der aufgeteilten Zielpopulation nach Zusatz des Führungsstrangs umfasst;
(b) Erstellen kombinatorischer Barcodes, um einem Ziel räumliche Positionskennungen zuzuordnen, wobei es sich bei dem Ziel um eine Oberfläche, ein Biomolekül oder ein biologisches Material handelt, wobei es sich bei dem biologischen Material um Biomoleküle, Zellen, Gewebe, Organe, Organoide, Vesikel, Liposomen oder Tröpfchen handelt;
(c) Bestimmen der Nähe von Biomolekülen und Erstellen von Aufzeichnungen, die Abstandsinformationen zwischen markierten Biomolekülen anzeigen; oder
(d) Erzeugen einer Nukleinsäuresequenz-Bibliothek.

## Revendications

1. Matrice d'acide nucléique comprenant :
(a) un premier brin guide comprenant dans la direction 3' à 5' une première région de synthèse, une deuxième région de synthèse, un premier domaine de jonction et une première région de blocage ; et
(b) un deuxième brin guide comprenant dans la direction 3' à 5' une deuxième région de blocage, un deuxième domaine de jonction et une troisième région de synthèse,
dans laquelle le premier domaine de jonction comprend une séquence nucléotidique sensiblement identique à une séquence nucléotidique de la troisième région de synthèse, et le premier domaine de jonction et le deuxième domaine de jonction sont sensiblement complémentaires l'un de l'autre et forment une région double brin, et
dans laquelle la première région de blocage et la deuxième région de blocage forment ensemble un premier domaine de blocage qui bloque l'activité de déplacement de brin d'une polymérase.

2. Matrice d'acide nucléique selon la revendication 1, dans laquelle :
(a) les première et deuxième régions de blocage sont liées l'une à l'autre de manière covalente ; et/ou
(b) la première ou la deuxième région de blocage comprend un segment de réticulation avec l'autre région de blocage pour former le premier domaine de blocage, éventuellement dans lequel le segment de réticulation comprend du 3-cyanovinylcarbozole.

3. Matrice d'acide nucléique selon la revendication 1, dans laquelle le deuxième brin guide comprend également une quatrième région de synthèse à l'extrémité 5' de la troisième région de synthèse ;
éventuellement dans laquelle le deuxième brin guide comprend également à son extrémité 5' dans la direction 3' à 5' un troisième domaine de jonction et une troisième région de blocage, et la matrice d'acide nucléique comprend également un troisième brin guide comprenant dans la direction 3' à 5' une quatrième région de blocage, un quatrième domaine de jonction et une cinquième région de synthèse,
dans laquelle le troisième domaine de jonction comprend une séquence nucléotidique sensiblement identique à une séquence nucléotidique de la cinquième région de synthèse,
dans laquelle les troisième et quatrième domaines de jonction sont sensiblement complémentaires l'un de l'autre et forment une région double brin, et
dans laquelle la troisième région de blocage et la quatrième région de blocage forment ensemble un deuxième domaine de blocage qui bloque l'activité de déplacement de brin d'une polymérase.

4. Matrice d'acide nucléique selon la revendication 3, dans laquelle :
(a) les troisième et quatrième régions de blocage sont reliées l'une à l'autre de manière covalente ; et/ou
(b) la troisième ou la quatrième région de blocage comprend un segment de réticulation avec l'autre région de blocage pour former le deuxième domaine de blocage, éventuellement dans lequel le segment de réticulation comprend du 3-cyanovinylcarbozole.

5. Matrice d'acide nucléique selon la revendication 3, dans laquelle le troisième brin guide comprend également une sixième région de synthèse à l'extrémité 5' de la cinquième région de synthèse ;
éventuellement dans laquelle le troisième brin guide comprend également à son extrémité 5' dans la direction 3' à 5' un cinquième domaine de jonction et une cinquième région de blocage, et la matrice d'acide nucléique comprend également un quatrième brin guide comprenant dans la direction 3' à 5' une sixième région de blocage, un sixième domaine de jonction et une septième région de synthèse,
dans laquelle le cinquième domaine de jonction comprend une séquence nucléotidique sensiblement identique à une séquence nucléotidique de la septième région de synthèse, et le cinquième domaine de jonction et le sixième domaine de jonction sont sensiblement complémentaires l'un de l'autre et forment une région double brin, et
dans laquelle la cinquième région de blocage et la sixième région de blocage forment ensemble un troisième domaine de blocage qui bloque l'activité de déplacement de brin d'une polymérase.

6. Matrice d'acide nucléique selon la revendication 5, dans laquelle :
(a) les cinquième et sixième régions de blocage sont reliées l'une à l'autre de manière covalente ; et/ou
(b) la cinquième ou la sixième région de blocage comprend un segment de réticulation avec l'autre région de blocage pour former le troisième domaine de blocage,
éventuellement dans lequel le segment de réticulation comprend du 3-cyanovinylcarbozole.

7. Matrice d'acide nucléique selon la revendication 5, dans laquelle le quatrième brin guide comprend également une huitième région de synthèse à l'extrémité 5' de la septième région de synthèse ;
éventuellement dans laquelle le quatrième brin guide comprend également à son extrémité 5' dans la direction 3' à 5' un septième domaine de jonction et une septième région de blocage, et la matrice d'acide nucléique comprend également un cinquième brin guide comprenant dans la direction 3' à 5' une huitième région de blocage, un huitième domaine de jonction et une neuvième région de synthèse,
dans laquelle le septième domaine de jonction comprend une séquence nucléotidique sensiblement identique à une séquence nucléotidique de la neuvième région de synthèse, et le septième domaine de jonction et le huitième domaine de jonction sont sensiblement complémentaires l'un de l'autre et forment une région double brin, et
dans laquelle la septième région de blocage et la huitième région de blocage forment ensemble un quatrième domaine de blocage qui bloque l'activité de déplacement de brin d'une polymérase.

8. Matrice d'acide nucléique selon la revendication 7, dans laquelle :
(a) les septième et huitième régions de blocage sont reliées l'une à l'autre de manière covalente ; et/ou
(b) la septième ou la huitième région de blocage comprend un segment de réticulation avec l'autre région de blocage pour former le quatrième domaine de blocage, éventuellement dans lequel le segment de réticulation comprend du 3-cyanovinylcarbozole.

9. Matrice d'acide nucléique selon l'une quelconque des revendications précédentes, dans laquelle :
(a) le segment de réticulation comprend du 3-cyanovinylcarbozole ;
(b) une ou plusieurs régions utilisent un code à trois lettres ; ou
(c) au moins l'un parmi le premier, le deuxième, le troisième ou le quatrième domaine de blocage comprend une région à double brin ou un étirement de monomère poly.

10. Matrice d'acide nucléique selon la revendication 1, dans laquelle la matrice d'acide nucléique ou au moins l'un des premier, deuxième, troisième ou quatrième domaines de blocage comprend une modification d'acide nucléique ;
éventuellement dans laquelle la modification d'acide nucléique est une nucléobase modifiée ou un fluorophore sur l'extrémité 3' et/ou 5'.

11. Matrice d'acide nucléique selon la revendication 1, dans laquelle la matrice d'acide nucléique :
(a) est conjuguée à un support solide ;
(b) comprend également une amorce recuite ou hybridée à l'un des brins guides ; ou
(c) comprend également une amorce recuite ou hybridée au premier brin guide.

12. Procédé de formation d'une matrice d'acide nucléique, le procédé comprenant le recuit ou l'hybridation d'un premier brin guide et d'un second brin guide, dans lequel :
(a) le premier brin guide comprend dans la direction 3' à 5' une première région de synthèse, une deuxième région de synthèse, un premier domaine de jonction et une première région de blocage ; et
(b) le deuxième brin guide comprend dans la direction 3' à 5' une deuxième région de blocage, un deuxième domaine de jonction et une troisième région de synthèse,
dans lequel le premier domaine de jonction comprend une séquence nucléotidique sensiblement identique à une séquence nucléotidique de la troisième région de synthèse, et le premier domaine de jonction et le deuxième domaine de jonction sont sensiblement complémentaires l'un de l'autre et forment une région double brin, et
dans lequel la première région de blocage et la deuxième région de blocage forment ensemble un domaine de blocage qui bloque l'activité de déplacement de brin d'une polymérase.

13. Procédé de synthèse d'une séquence d'acide nucléique, le procédé comprenant :
(a) la fourniture ou l'obtention d'une matrice d'acide nucléique selon l'une quelconque des revendications 1 à 11 ;
(b) le recuit ou l'hybridation d'une amorce, si elle n'a pas déjà été recuite, avec la matrice d'acide nucléique ; et
(c) l'extension d'une séquence nucléotidique à l'aide d'une ADN polymérase ayant une activité de déplacement de brin à partir de l'extrémité 3'-terminale de l'amorce ;
éventuellement, dans lequel le procédé comprend également l'amplification de la séquence d'acide nucléique ou comprend également le séquençage de la séquence d'acide nucléique synthétisée.

14. Procédé de synthèse d'une séquence d'acide nucléique, le procédé comprenant :
(a) le recuit ou l'hybridation d'une amorce, si elle n'a pas déjà été recuite, avec un premier brin guide, dans lequel le premier brin guide comprend dans une direction 3' à 5' une première région de synthèse, une deuxième région de synthèse, un domaine de jonction et une région de blocage ; et
(b) l'extension d'une séquence nucléotidique à l'aide d'une ADN polymérase ayant une activité de déplacement de brin à partir de l'extrémité 3'-terminale de l'amorce ;
(c) l'ajout d'un deuxième brin guide à la réaction de synthèse, le second brin guide comprenant dans une direction 3' à 5' une région de blocage, un domaine de jonction, une première région de synthèse, une deuxième région de synthèse, un deuxième domaine de jonction et une deuxième région de blocage, dans lequel le premier domaine de blocage du second brin guide et le domaine de blocage du premier brin guide forment ensemble un domaine de blocage qui bloque l'activité de déplacement de brin d'une polymérase, le premier domaine de jonction du deuxième brin guide comprend une séquence nucléotidique sensiblement complémentaire à une séquence d'acide nucléique du domaine de jonction du premier brin guide pour former une région à double brin, et la première région de synthèse du deuxième brin guide comprend une séquence nucléotidique sensiblement identique à une séquence d'acide nucléique du domaine de jonction du premier brin guide ;
(d) éventuellement, la réticulation du premier domaine de blocage du deuxième brin guide et du domaine de blocage du premier brin guide ;
(e) l'extension supplémentaire de la séquence nucléotidique ;
(f) éventuellement, l'ajout de nouveaux brins guides supplémentaires à la réaction de synthèse et l'extension supplémentaire de la séquence nucléotidique avant l'ajout de chaque nouveau brin guide supplémentaire, dans lequel chaque brin guide supplémentaire comprend dans une direction 3' à 5' une région de blocage, un domaine de jonction, une première région de synthèse, une deuxième région de synthèse, un deuxième domaine de jonction et une deuxième région de blocage, dans lequel la première région de blocage du nouveau brin guide et la deuxième région de blocage du dernier brin guide ajouté à la réaction de synthèse forment ensemble un domaine de blocage qui bloque l'activité de déplacement de brin d'une polymérase ; le premier domaine de jonction du nouveau brin guide comprend une séquence nucléotidique sensiblement complémentaire à une séquence d'acide nucléique du deuxième domaine de jonction du dernier brin guide ajouté à la réaction de synthèse pour former une région double brin ; et la première région de synthèse du nouveau brin guide comprend une séquence nucléotidique sensiblement identique à une séquence d'acide nucléique du deuxième domaine de jonction du dernier brin guide ajouté à la réaction de synthèse ;
(g) éventuellement, la réticulation de la première région de blocage du nouveau brin guide et de la deuxième région de blocage du dernier brin guide ajouté à la réaction de synthèse avant l'extension supplémentaire de la séquence nucléotidique ;
éventuellement comprenant également :
(h) l'ajout d'un brin guide terminal à la réaction de synthèse, où le brin guide terminal comprend dans une direction 3' à 5' une région de blocage, un domaine de jonction, une première région de synthèse, une deuxième région de synthèse, un deuxième domaine de jonction et une deuxième région de blocage, dans lequel la première région de blocage du brin guide terminal et la deuxième région de blocage du dernier brin guide ajouté à la réaction de synthèse forment ensemble un domaine de blocage qui bloque l'activité de déplacement de brin d'une polymérase ; le premier domaine de jonction du brin guide terminal comprend une séquence nucléotidique sensiblement complémentaire à une séquence d'acide nucléique du deuxième domaine de jonction du dernier brin guide ajouté à la réaction de synthèse pour former une région double brin ; et la première région de synthèse du brin guide terminal comprend une séquence nucléotidique sensiblement identique à une séquence d'acide nucléique du deuxième domaine de jonction du dernier brin guide ajouté à la réaction de synthèse ;
(i) éventuellement, la réticulation du premier domaine de blocage du brin guide terminal et du domaine de blocage du dernier brin guide ajouté à la réaction ; et
(j) l'extension supplémentaire de la séquence nucléotidique ; éventuellement, dans lequel le procédé comprend également l'amplification de la séquence d'acide nucléique ou comprend également le séquençage de la séquence d'acide nucléique synthétisée.

15. Utilisation d'une matrice d'acide nucléique selon l'une quelconque des revendications 1 à 11 pour :
(a) créer des codes à barres combinatoires pour attribuer des identités uniques à une population cible, dans laquelle la population cible est constituée de positions/partitions de surface, de matrices, de biomolécules, de bibliothèques moléculaires ou d'un matériau biologique, éventuellement dans laquelle le matériau biologique est constitué de vésicules, de cellules, de tissus, d'organoïdes, de gouttelettes, de liposomes, de petites molécules ou de billes, et éventuellement l'utilisation comprenant la division de la population cible avant l'ajout d'un brin guide et la mise en commun de la population cible divisée après l'ajout du brin guide ;
(b) créer des codes à barres combinatoires pour attribuer des identifiants de position spatiale à une cible, dans laquelle la cible est une surface, une biomolécule ou un matériau biologique, éventuellement dans laquelle le matériau biologique est constitué de biomolécules, de cellules, de tissus, d'organes, d'organoïdes, de vésicules, de liposomes ou de gouttelettes ;
(c) déterminer la proximité de biomolécules et créer des enregistrements indiquant des informations de distance entre les biomolécules marquées ; ou
(d) générer une bibliothèque de séquences d'acide nucléique.
